(19) Europäisches Patentamt
European Patent Office
Office européen des brevets

(11) EP 0 829 478 A2

# (12) EUROPEAN PATENT APPLICATION

(43) Date of publication:
18.03.1998 Bulletin 1998/12

(21) Application number: 97115526.2

(22) Date of filing: 08.09.1997

(51) Int. Cl.$^6$: C07D 405/06, A61K 31/41, C07D 405/14, A61K 31/44, A61K 31/415, C07D 233/60, C07D 249/08, C07D 417/06, A61K 31/425, C07D 405/12, A61K 31/50, C07D 407/06

(84) Designated Contracting States:
AT BE CH DE DK ES FI FR GB GR IE IT LI LU MC NL PT SE
Designated Extension States:
AL LT LV RO SI

(30) Priority: 09.09.1996 EP 96114390
18.08.1997 EP 97114246

(71) Applicant:
F. HOFFMANN-LA ROCHE AG
4070 Basel (CH)

(72) Inventors:
- Ichihara, Shigeyasu
Kanagawa-ken (JP)
- Murasaki, Chikako
Kanagawa-ken (JP)
- Ohga, Noriko
Yokohama-shi, Kanagawa-ken (JP)
- Ohwada, Jun
Kamakura-shi, Kanagawa-ken (JP)
- Sawada, Daisuke
Tokyo-to (JP)
- Shimma, Nobuo
Kanagawa-ken (JP)
- Shirai, Michio
Fujisawa-shi, Kanagawa-ken (JP)
- Umeda,Isao,
Imp.Higashihakuraku Gar.House Rm B-513
Yokohama-shi, Kanagawa-ken (JP)

(74) Representative: Mahé, Jean et al
F.Hoffmann-La Roche AG
Patent Department (PLP),
124 Grenzacherstrasse
4070 Basel (CH)

# (54) N-Benzylimidazolium and N-benzyltriazolium derivatives, their preparation and their use as antifungal and antimycotic agents

(57) N-Benzylazolium derivatives of the general formula (I),

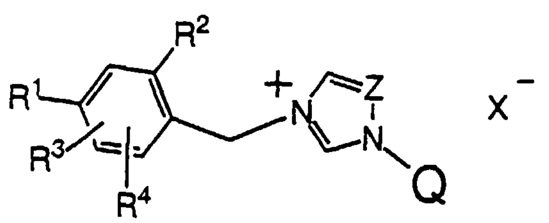

(I)

wherein

Q is the remainder of an azole compound of the formula II

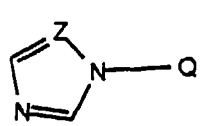

(II)

possessing antifungal activity;



Printed by Xerox (UK) Business Services
2.15.12/3.4

(Cont. next page)

| | |
|---|---|
| Z | is nitrogen or methine; |
| $R^1$ and $R^2$ | are each independently a hydrogen atom or a group -OY [in which Y is a group easily hydrolyzable under physiological condition]; |
| $R^3$ and $R^4$ | are each independently a hydrogen or halogen atom, lower alkyl, lower alkoxy, lower alkylthio, (lower alkylcarbonyl)thiomethyl, carboxy or methoxycarbonyl; and |
| $X^-$ | is a pharmaceutically acceptable anion, |

as well as salts, hydrates or solvates of the compounds of the general formula (I) have antifungal properties.

## Description

Although several azole compounds are currently used for systemic mycoses, none of them fulfills the necessary clinical requirement in full extent, i.e. efficacy against major systemic mycoses including disseminated aspergillosis, safety, and oral or parenteral formulations. Particularly, demand of a parenteral administration of the azole compounds is increasing for the treatment of serious systemic mycoses. Most of the azole compounds on the market as well as under development are highly lipophilic molecules that make the parenteral formulation difficult.

The present invention relates to novel water soluble azole compounds useful for the treatment of systemic mycoses and suitable for both oral and particularly parenteral administration, a process for their manufacture, antifungal compositions containing them and a method for treating mycoses.

More particularly, the present invention relates to novel azole compounds represented by the general formula (I),

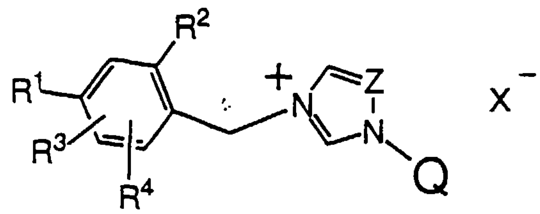

(I)

wherein

Q is the remainder of an azole compound of the formula II

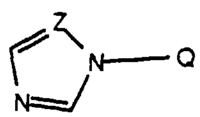

(II)

possessing antifungal activity;

| | |
|---|---|
| Z | is nitrogen or methine; |
| $R^1$ and $R^2$ | are each independently a hydrogen atom or a group -OY [in which Y is a group easily hydrolyzable under physiological condition]; |
| $R^3$ and $R^4$ | are each independently a hydrogen or halogen atom, lower alkyl, lower alkoxy, lower alkylthio, (lower alkylcarbonyl)thiomethyl, carboxy or methoxycarbonyl; and |
| $X^-$ | is a pharmaceutically acceptable anion, |

as well as salts, hydrates or solvates of the compounds of the general formula (I).

Preferred among the above azole compounds of formula I are those of formula (I'),

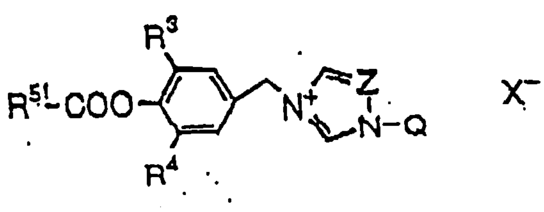

(I')

wherein

| | |
|---|---|
| $R^{5'}$ | in a straight-chain or branched $C_1$-$C_5$ alkyl, aryl, pyridyl, pyrrolidinyl or a group A-NH-B-(wherein A is a hydrogen atom or a straight-chain or branched $C_1$-$C_5$ alkyl; B is a straight-chain or branched $C_1$-$C_4$ alkylene, $-CH_2-CONH-CH_2-$ or $-CH_2CH_2CH_2-CH(NH_2)-$); |

$R^3$ and $R^4$ are each independently a hydrogen or halogen atom or a lower alkoxy; and
Z, Q and X $^-$ are as defined above.

Especially preferred among the above azole compounds of formulae I and I' are those wherein

Q is the group of the formula,

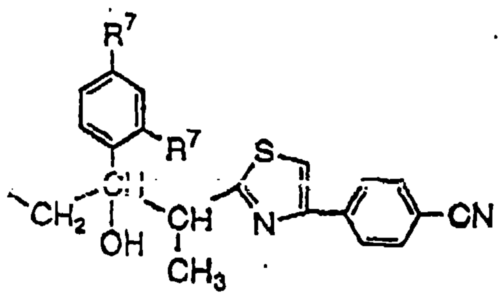

(Q1),

or

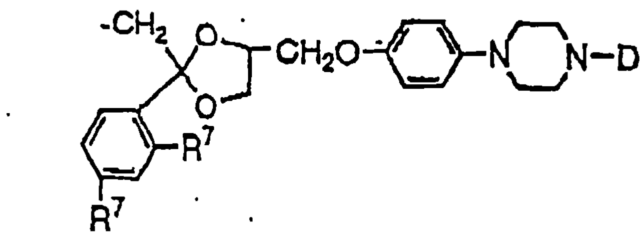

(Q2),

wherein

D is a lower alkanoyl or the group of the formula,

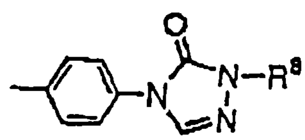

$R^7$ is a halogen atom, and
$R^8$ is a straight-chain or branched $C_1$-$C_4$ alkyl.

The respective groups in the general formula (I) which are defined above are explained in more detail as follows: The Example of azole compounds of the formula II

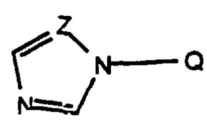

(II)

are

dl-1-[2-(2,4-dichlorophenyl)-2-[(2,4-dichlorophenyl)methoxy]ethyl]-1H-imidazole,
dl-cis-1-acetyl-4-[4-[2-(2,4-dichlorophenyl)-2-(imidazol-1-ylmethyl)-1,3-dioxolan-4-yl]methoxy]phenyl]piperazine,
dl-2-[(RS)-sec-butyl]-4-[4-[4-[4-[(2R,4S)-2-(2,4-dichlorophenyl)-2-(1H-1,2,4-triazol-1-ylmethyl)-1,3-dioxolan-4-yl]methoxy]phenyl]-1-piperazinyl]phenyl-3H-[1,2,4]triazol-3-one
2-[(1R,2R)-2-(2,4-diflorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone,
(+)-2-(2,4-difluorophenyl)-3-methyl-1-(1H-1,2,4-triazol-1-yl)-3-(6-(1H-1,2,4-triazol-1-yl)pyridazin-3-ylthio)butan-2-ol,
(2R)-2-(2,4-difluorophenyl)-1-[3-[(E)-4-(2,2,3,3-tetrafluoropropoxy)styryl]-(1,2,4-triazol-1-yl)-3-(1,2,4-triazol-1-yl)]propan-2-ol
dl-threo-2-(2,4-difluorophenyl)-3-methyl-sulfonyl-1-(1H-1,2,4-triazol-1-yl)-butan-2-ol,
(-)-4-[4-[4-[4-[[5-(2,4-difluorophenyl)-5-(1H-1,2,4-triazol-1-ylmethyl)tetrahydrofuran-3-yl]methoxy]phenyl]piperazinyl]phenyl]-2-[(1S,2S)-1-ethyl-2-hydroxyropyl]-3H-1,2,4-triazol-3-one
(2R,3R)-3-[4-(4-cyanophenyl)thiazol-2-yl)]-2-(2,4-difluorophenyl)-1-(1H-1,2,4-triazol-1-yl)-butan-2-ol,
3-methyl-3-methylthio-1-(1,2,4-triazol-1-yl)-2-(trifluoromethylphenyl)-butan-2-ol,
1-[[(1R,2S,6R)-2-methoxy-3,3-dimethyl-6-(2-*p*-tolylethyl)cyclohexyl]methyl]-1H-[1,2,4]triazol,
(5R,6R)-2,2-dimethyl-6-[(1H-1,2,4-triazol-1-yl)methyl]-5-[[4-(trifluoromethoxy)phenoxy]methyl]cyclohexanone O-methyloxime, and the like.

$R^1$ and $R^2$ are each independently a hydrogen atom or a group -OY [in which Y is an easily hydrolyzable radical under physiological condition].

In the above a group Y which is easily hydrolyzable under physiological condition means preferably the acyl residue of an amino acid or a group represented by the formula,

$$R^5CO\text{-}, \text{ or } (R^6O)_2PO\text{-}$$

wherein $R^5$ is hydrogen, lower alkoxy, lower alkyl which may be optionally substituted with carboxyl, amino, lower alkyl amino, dilower alkyl amino, or aryl; and $R^6$ is hydrogen or lower alkyl.

As used in this specification, the term „lower alkyl“ refers to preferably straight or branched chain having 1 to 4 carbon atoms such as methyl, ethyl, n-propyl, iso-propyl, butyl, iso-butyl or tert-butyl.

The term „lower alkoxy“ refers to preferably straight or branched chain having 1 to 4 carbon atoms such as methoxy, ethoxy, n-propoxy, iso-propoxy, n-butoxy, iso-butoxy.

The term „aryl“ means preferably an unsubstituted or substituted aryl radical such as phenyl, methoxyphenyl, pyridyl, pyrazinyl or furyl. Preferably, Y is formyl, acetyl, propionyl, isobutyryl, pivaloyl, succinoyl, benzoyl, nicotinoyl, phosphoryl, dimethylphosphoryl, aminoacetyl, 3-aminopropionyl, 4-aminobutyryl, (2-amino-acetylamino)-acet, (S)-2,5-diaminopentoyl, (S)-2-aminopropionyl, (S)-pyrrolidine-2-carbonyl, (methylamino)acetyl, (propylamino)acetyl, (S)-2-(methylamino)propionyl, 3-(methylamino)propionyl, (S)-2-amino-3-methylbutanoyl, (isopropylamino)acetyl, (2S)-2-(ethylamino)propionyl, (ethylamino)acetyl and the like.

$R^3$ and $R^4$ are each independently a hydrogen or halogen atom, lower alkyl, lower alkoxy, lower alkylthio, (lower alkylcarbonyl)thiomethyl, carboxy or methoxycarbonyl.

The term „halogen“ denotes fluorine, chlorine or bromine.

The term „lower alkylthio“ refers to preferably straight or branched chain having 1 to 4 carbon atoms such as methylthio, ethylthio, n-propylthio.

The term „lower alkyl“ and "lower alkoxy" are the same as described above.

Preferably, $R^3$ and $R^4$ are independently methyl, methoxy, or chlorine.

$X^-$ is an anion from a pharmaceutically acceptable inorganic acid, e.g. a mineral acid; such as chloride, bromide or sulfate; or from an organic acid, e.g. an aliphatic, aromatic or araliphatic carboxylic or sulfonic acid such as acetoxy, trifluoroacetoxy, mesyloxy anion and the like.

Particularly preferred compounds in accordance with the present invention are:

dl-1-(4-acetoxy-3,5-dimethylbenzyl)-3-[2-(2,4-dichlorobenzyloxy)-2-(2,4-dichlorophenyl)ethyl]-3H-imidazol-1-ium bromide,
dl-1-(4-acetoxy-3-methylbenzyl)-3-[2-(2,4-dichlorobenzyloxy)-2-(2,4-dichlorophenyl)ethyl]-3H-imidazol-1-ium bromide,
dl-1-(4-acetoxybenzyl)-3-[2-(2,4-dichlorobenzyloxy)-2-(2,4-dichlorophenyl)ethyl]-3H-imidazol-1-ium bromide,
dl-1-(4-acetoxybenzyl)-3-[(2R,4S)-4-[4-(4-acetylpiperazin-1-yl)phenoxymethyl]-2-(2,4-dichlorophenyl)-[1,3]dioxan-2-ylmethyl]-3H-imidazol-1-ium bromide,
dl-1-(4-acetoxy-3,5-dimethylbenzyl)-3-[(2R,4S)-4-[4-(4-acetylpiperazin-1-yl)phenoxymethyl]-2-(2,4-dichlorophenyl)-[1,3]dioxan-2-ylmethyl]-3H- imidazol-1-ium bromide,
dl-1-(4-acetoxy-3-methylbenzyl)-3-[(2R,4S)-4-[4-(4-acetylpiperazin-1-yl)phenoxymethyl]-2-(2,4-dichlorophenyl)-[1,3]dioxan-2-ylmethyl]-3H-imidazol-1-ium bromide,

dl-1-(4-acetoxy-3-methoxybenzyl)-3-[(2R,4S)-4-[4-(4-acetylpiperazin-1-yl)phenoyxmethyl]-2-(2,4-dichlorophenyl)-[1,3]dioxan-2-ylmethyl]-3H-imidazol-1-ium bromide,

dl-3-[(2R,4S)-4-[4-(4-acetylpiperazin-1-yl)phenoxymethyl]-2-(2,4-dichlorophenyl)-[1,3]dioxan-2-ylmethyl]-1-(4-isobutyryloxy-3,5-dimethylbenzyl)-3H-imidazol-1-ium bromide,

dl-3-[(2R,4S)-4-[4-(4-acetylpiperazin-1-yl)phenoxymethyl]-2-(2,4-dichlorophenyl)-[1,3]dioxan-2-ylmethyl]-1-(4-pivaloyloxy-3,5-dimethylbenzyl)-3H-imidazol-1-ium bromide,

dl-3-[(2R,4S)-4-[4-(4-acetylpiperazin-1-yl)phenoxymethyl]-2-(2,4-dichlorophenyl)-[1,3]dioxan-2-ylmethyl]-1-[4-(2,2-dimethylpropionyloxy)benzyl]-3H-imidazol-1-ium bromide,

dl-4-(4-benzoyloxy-3,5-dimethylbenzyl)-1-[4-[4-[4-[4-(1-(2-butyl-5-oxo-1,5-dihydro-[1,2,4]triazol-4-yl)phenyl]piperazin-1-yl]phenoxymethyl]-2-(2,4-dichlorophenyl)-[1,3]dioxolan-2-ylmethyl]-1H-[1,2,4]triazol-4-ium bromide,

di-4-[4-(pyridine-3-carbonyloxy)-3,5-dimethylbenzyl]-1-[4-[4-[4-[4-(1-(2-butyl-5-oxo-1,5-dihydro-[1,2,4]triazol-4-yl)phenyl]piperazin-1-yl]phenoxymethyl]-2-(2,4-dichlorophenyl)-[1,3] dioxolan-2-ylmethyl]-1H-[1,2,4]triazol-4-ium bromide,

dl-4-(4-acetoxy-3,5-dimethylbenzyl)-1-[4-[4-[4-[4-[1-(2-butyl-5-oxo-1,5-dihydro-[1,2,4]triazol-4-yl)phenyl]piperazin-1-yl]phenoxymethyl]-2-(2,4-dichlorophenyl)-[1,3]dioxolan-2-ylmethyl]-1H-[1,2,4]triazol-4-ium bromide,

dl-4-(4-acetoxy-3-methylbenzyl)-1-[4-[4-[4-[4-[1-(2-butyl-5-oxo-1,5-dihydro-[1,2,4]triazol-4-yl)phenyl]piperazin-1-yl]phenoxymethyl]-2-(2,4-dichlorophenyl)-[1,3]dioxolan-2-ylmethyl]-1H-[1,2,4]triazol-4-ium bromide,

di-4-(4-acetoxybenzyl)-1-[4-[4-[4-[4-[1-(2-butyl-5-oxo-1,5-dihydro-[1,2,4]triazol-4-yl)phenyl]piperazin-1-yl]phenoxymethyl]-2-(2,4-dichlorophenyl)-[1,3]dioxolan-2-ylmethyl]-1H-[1,2,4]triazol-4-ium bromide,

dl-1-[4-(4-[4-[4-(1-sec-butyl-5-oxo-1,5-dihydro-[1,2, 4]triazol-4-yl)phenyl]-piperazin-1-yl]-phenoxymethyl)-2-(2,4-dichloro-phenyl)-[1,3]dioxolan-2-ylmethyl]-4-(4-hexanoyloxy-3,5-dimethylbenzyl)-1H-[1,2,4]triazol-4-ium methanesulfonate,

4-(4-acetoxy-3,5-dimethylbenzyl)-1-[(2R,3R)-2-(2,4-difluorophenyl)-2-hydroxy-3-{5-oxo-4-[4-(2,2,3,3-tetrafluoropropoxy)phenyl]-4,5-dihydro- [1,2,4]triazol-1-yl}butyl]-1H-[1,2,4]triazol-4-ium bromide,

4-(4-acetoxy-3,5-dimethylbenzyl)-1-[(2R)-2-(2,4-difluorophenyl)-2-hydroxy-3-methy]-3-(6-[1,2,4]triazol-1-yl-pyridazin-3-ylsulfanyl)butyl]-1H-[1,2,4]triazol-4-ium bromide,

4-(4-acetoxy-3,5-dimethylbenzyl)-1-[(2R)-2-(2,4-difluorophenyl)-2-hydroxy-3-(3-{(Z)-2-[4-(2,2,3,3-tetrafluoropropoxy)phenyl]vinyl}-[1,2,4]triazol-1-yl)propyl]-1H-[1,2,4]triazol-4-ium bromide,

4-(4-acetoxy-3,5-dimethylbenzyl)-1-[(2R,3R)-2-(2,4-difluorophenyl)-2-hydroxy-3-methanesulfonylbutyl]-1H-[1,2,4]triazol-4-ium bromide,

4-(4-acetoxy-3,5-dimethylbenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,

4-(4-acetoxy-3-methylbenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,

4-(4-acetoxybenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,

4-(4-acetoxy-3,5-dichlorobenzy)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,

4-(4-acetoxy-3-chlorobenzyl)-1-[(2R-cis)-2-(2,4-difluorophenyl)-4-[4-[4-[4-[1-[(1S,2S)-1-(ethyl-2-hydroxypropyl)]-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,

4-(4-acetoxy-3,5-dimethylbenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,

4-(4-acetoxy-3-methylbenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,

4-(4-acetoxybenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,

4-(4-acetoxy-3,5-dichlorobenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,

4-(4-acetoxy-3-chlorobenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,

(2R,3R)-4-(4-aminoacetoxy-2-carboxybenzyl)-1-[3-[4-(4-cyano-phenyl)thiazol-2-yl]-2-(2,4-difluorophenyl)-2-hydroxybutyl]-1H-[1,2,4]triazol-4-ium bromidetrifluoroacetic acid salt,

1-[(2R,3R)-3-[4-(4-cyanophenyl)-thiazol-2yl]-2-(2,4-difluorophenyl)-2-hydroxybutyl]-4-[(S)-3,5-dimethyl-4-(pyrrolidine-2-carbonyloxy)-benzyl]-1H-[1,2,4]triazol-4-ium bromide trifluoroacetic acid salt,
(2R,3R)-4-(4-aminoacetoxy-3,5-dimethylbenzyl)-1-[3-[4-(4-cyanophenyl)-thiazol-2-yl]-2-(2,4-difluorophenyl)-2-hydroxybutyl]-1H-[1,2,4]triazol-4-iumbromide trifluoroacetic acid salt,
(2R,3R)-4-[4-(3-aminopropionyloxy)-3,5-dimethylbenzyl]-1-[4-[4-(4-cyanophenyl)-thiazol-2-yl]-2-(2,4-difluorophenyl)-2-hydroxy-3-methylbutyl]-1H-[1,2,4]triazol-4-ium bromide trifluoroacetic acid salt,
(2R,3R)-4-[4-(4-amino-butyryloxy)-3,5-dimethylbenzyl]-1-[4-[4-(4-cyano-phenyl)-thiazol-2-yl]-2-(2,4-difluorophenyl)-2-hydroxy-3-methylbutyl]-1H-[1,2,4]triazol-4-ium bromide trifluoroacetic acid salt,
(2R,3R)-4-[4-[[2-(aminoacetyl)amino]acetoxy]-3,5-dimethylbenzyl]-1-[3-[4-(4-cyanophenyl)-thiazol-2-yl]-2-(2,4-difluorophenyl)-2-hydroxy-butyl]-1H-[1,2,4]triazol-4-ium bromide trifluoroacetic acid salt,
1-[(2R,3R)-3-[4-(4-cyanophenyl)-thiazol-2-yl]-2-(2,4-difluorophenyl)-2-hydroxybutyl]-4-[4-[(S)-2,5-diaminopentoyloxy]-3,5-dimethyl-benzyl]-1H-[1,2,4]triazol-4-ium bromide trifluoroacetic acid salt,
4-[4-[(S)-2-amino-propionyloxy)-3,5-dimethylbenzyl]-1-[(2R,3R)-3-[4-(4-cyanophenyl)-thiazol-2-yl]-2-(2,4-difluorophenyl)-2-hydroxy-butyl]-1H-[1,2,4]triazol-4-ium bromide trifluoroacetic acid salt,
1-[(2R,3R)-3-[4-(4-cyanophenyl)-thiazol-2-yl]-2-(2,4-difluorophenyl)-2-hydroxybutyl]-4-[3,5-dimethyl-4-[(methylamino)acetoxy]benzyl]-1H-[1,2,4]triazol-4-ium bromide trifluoroacetic acid salt,
(2R,3R)-1-[3-[4-(4-cyanophenyl)-thiazol-2-yl]-2-(2,4-dlfluorophenyl)-2-hydroxybutyl]-4-[3,5-dimethyl-4-[(propylamino)acetoxy]benzyl]-1H-[1,2,4]triazol-4-ium bromide trifluoroacetic acid salt,
1-[(2R,3R)-3-[4-(4-cyanophenyl)-thiazol-2-yl]-2-(2,4-difluorophenyl)-2-hydroxybutyl]-4-[4-[(S)-2-(methylamino)propionyloxy]-3,5-dimethyl-benzyl]- 1H-[1,2,4]triazol-4-ium bromide trifluoroacetic acid salt,
(2R,3R)-1-[4-[4-(4-cyanophenyl)-thiazol-2-yl]-2-(2,4-difluorophenyl)-2-hydroxy-3-methyl-butyl]-4-[3,5-dimethyl-4-[3-(methylamino)propionyloxy]benzyl]-1H-[1,2,4]triazol-4-ium bromide trifluoroacetate,
4-[4-[(S)-2-amino-3-methyl-butanoyloxyl-3,5-dimethylbenzyl]-1-[(2R,3R)-3-[4-(4-cyanophenyl)-thiazol-2-yl]-2-(2,4-difluorophenyl)-2-hydroxybutyl]-1H-[1,2,4]triazol-4-ium bromide trifluoroacetic acid salt,
(2R,3R)-1-[3-[4-(4-cyanophenyl)-thiazol-2-yl]-2-(2,4-difluorophenyl)-2-hydroxy-butyl]-4-[4-[(isopropylamino)acetoxy]-3,5-dimethylbenzyl)-1H-[1,2,4]triazol-4-ium bromide trifluoroacetic acid salt,
1-[(2R,3R)-3-[4-(4-cyanophenyl)-thiazol-2-yl]-2-(2,4-difluorophenyl)-2-hydroxybutyl]-4-[(2S)-4-[2-(ethylamino)propionyloxy]-3,5-dimethyl-benzyl]-1H-[1,2,4]triazol-4-ium bromide trifluoroacetic acid salt,
(2R,3R)-1-[3-[4-(4-cyanophenyl)-thiazol-2-yl]-2-(2,4-difluorophenyl)-2-hydroxybutyl]-4-[4-[(ethylamino)acetoxy]-3,5-dimethylbenzyl]-1H-[1,2,4]triazol-4-ium bromide trifluoroacetic acid salt
4-(4-acetoxy-3,5-dimethylbenzyl)-1-[2-hydroxy-3-methyl-3-methylsulfanyl-2-(4-trifluoromethylphenyl)butyl]-1H-[1,2,4]triazol-4-ium bromide,
4-(4-acetoxy-3,5-dimethylbenzyl)-1-[[(1R,6R)-2-methoxyimino-3,3-dimethyl-6-[(4-trifluoromethoxyphenoxy)methyl]cyclohexyl]methyl]-1H-[1,2,4]triazol-4-ium bromide,
4-(4-acetoxy-3,5-dimethylbenzyl)-1-[(1R,2S,6R)-2-methoxy-3,3-dimethyl-6-(2-*p*-tolyl-ethyl)cyclohexylmethyl]-1H-[1,2,4]triazol-4-ium bromide,
1-[2R,3R)-3-[4-(4-cyano-phenyl)-thiazol-2-yl]-2-(2,4-difluoro-phenyl)-2-hydroxy-butyl]-4-(3,5-dimethyl-4-methylaminoacetoxy-benzyl)-1H-[1,2,4]triazol-4-ium bromide hydrochloric acid salt,
1-[2R,3R)-3-[4-(4-cyano-phenyl)-thiazol-2-yl]-2-(2,4-difluoro-phenyl)-2-hydroxy-butyl]-4-(3,5-dimethyl-4-methylaminoacetoxy-benzyl)-1H-[1,2,4]triazol-4-ium chloride hydrochloric acid salt, and
1-[2R,3R)-3-[4-(4-cyano-phenyl)-thiazol-2-yl]-2-(2,4-difluoro-phenyl)-2-hydroxy-butyl]-4-(3,5-dimethyl-4-methylaminoacetoxy-benzyl)-1H-[1,2,4]triazol-4-ium bromide hydrobromic acid salt.

The novel azole compounds represented by the general formula (I) as well as salts, hydrates or solvates thereof can be manufactured

A) by reacting an azole compound possessing antifungal activity, of the general formula (II)

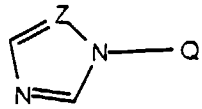 (II)

with a compound of the general formula (III),

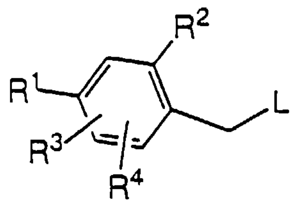

(III)

wherein $R^1$ and $R^2$ are each independently a hydrogen atom or a group-OY [in which Y is a group easily hydrolyzable under physiological conditions]; $R^3$ and $R^4$ are each independently a hydrogen or halogen atom, lower alkyl, lower alkoxy, lower alkylthio, (lower alkylcarbonyl) thiomethyl, carboxy or methoxycarbonyl; and L is a leaving group; or

B) by reacting a compound of the general formula (II) as defined above with a compound of the general formula (III) wherein one of $R^1$ or $R^2$ is hydroxy, followed by reaction of the resulting compound with a compound of formula $R^5COL$, or $(R^6O)_2POL$, wherein $R^5CO$ is the acyl residue of a N-protected amino acid, or $R^5$ is hydrogen, lower alkoxy, lower alkyl which may be optionally substituted with carboxyl, amino, lower alkyl amino with or without protecting group, di-lower alkyl amino, or aryl; and $R^6$ is hydrogen or lower alkyl, and L is a leaving group such as hydroxy, chlorine, bromine, $OCOR^5$, methanesulfonyl, p-toluenesulfonyl and the like.

The reaction of the compound of the general formula (II) with the compound of the general formula (III) can be carried out in a solvent such as methylene chloride, chloroform, benzene, toluene, acetonitrile, tetrahydrofuran, dioxane, or dimethylformamide, preferably chloroform, acetonitrile, or dimethylformamide.

The reaction time in the above benzylation reaction may be varied within a relatively wide range. In general, the reaction can be carried out at a temperature between 0°C and 100°C, preferably between 0°C and 50°C.

In process B, the acylation or phosphorylation can be performed with either free acid in the presence of a condensation agent such as 1-(3-dimethylaminopropyl)-3-ethylcarbodiimide hydrochloride and the like, or acid halides, acid anhydrides, mixed anhydrides, alkoxycarbonyl halides, dialkoxy phosphoryl chloride or phosphoryl chloride in the presence of an acid acceptor such as triethylamine, pyridine, picoline, lutidine, dimethylaminopyridine or alkali metal carbonate by the method known to those skilled in the art.

Preferably, an amino group present in $R^5$ in the compound of formula III is protected by a suitable amino protecting group as tert.-butoxy carbonyl.

The protecting group may, if necessary, be removed after the reaction by procedures known to those skilled in the art.

The compounds of formula I may contain an amino acid ester substituent $R^1$ and/or $R^2$ which substituents may form acid addition salts. The term "salts of compounds of formula I" refers to such acid addition salts. These salts may be derived from pharmaceutically acceptable acids as described earlier with reference to the Symbol $X^-$. The salt formation can be performed when removing a protecting group, or can be performed ad hoc by procedures known per se.

The hydration can be effected in the course of the manufacturing process or can occur gradually as a result of hygroscopic properties of an initially anhydrous product. Solvates with pharmaceutically acceptable solvents such as ethanol can be obtained for example, during crystallization.

The novel azole compounds represented by the general formula (I) as well as hydrates or solvates thereof have much higher water solubility than known antimycotic azole compounds represented by the general formula (II) (see table 1).

Table 1

| Compound (Example No.) | solubility (mg/ml) | solvent* |
|---|---|---|
| 1 | 1.0 | b |
| 2 | 0.4 | b |
| 3 | 0.4 | b |
| 4 | >2.0 | a |
| 5 | >1.0 | a |
| 6 | 6.5 | a |
| 7 | >1.0 | a |
| 8 | 14.0 | a |
| 9 | >2.0 | a |
| 10 | >2.0 | a |
| 11 | 6.0 | a |
| 12 | 5.0 | a |
| 13 | 0.5 | b |

* solvent a = distilled water , solvent b = Mellvaine buffer(pH8.02)

In addition, the novel azole compounds are chemically stable in aqueous solution at room temperature more than three days, but are efficiently converted into compounds of formula (II) in either mouse, rat, monkey or human plasma.

The conversion of representatives of the new azole compounds of the general formula (I) to ketoconazole and itraconazole, respectively, in human plasma are shown in table 2. The compounds of formula I were incubated with human plasma at a concentration of 10μg/ml at 37°C for up to 120 min.

Table 2

| Conversion of the new new azole compounds to ketoconazole (KCZ) and itraconazole (ICZ) in human plasma | | | | |
|---|---|---|---|---|
| Example No. | Conversion half-life (min) | Incubation time (min) | Observed (%) | |
| | | | Comp. I | KCZ |
| 9 | < 1 | 5 | < 5 | 89 |
| 11 | < 1 | 5 | < 5 | 100 |
| 4 | 8.4 | 20 | 19 | 74 |
| 10 | 3.5 | 10 | < 5 | 80 |
| | | | | ICZ |
| 1 | 53 | 60 | 47 | 28 |

In vivo efficacy of the compounds of the present invention is shown in Table 3. Male Fisher rats, strain F344/DuCrj, were employed for experimental infection models such as systemic candidiasis, systemic aspergillosis and pulmonary aspergillosis model. Immunocompetent 4 weeks old rats were used for systemic candidiasis or systemic aspergillosis which occurred her infection with Candida albicans conidia of $5x10^6$/rat or with Aspergillus fumigatus conidia of $6x10^5$/rat via tail vein. Otherwise for pulmonary aspergillosis model, rats had been immunosuppressed with cortisone acetate treatments prior to infection with $2x10^5$/rat intratrachially. Treatments were given twice on the first day and once

daily on following 4 days both for systemic and pulmonary aspergillosis (lb.i.+4q.d.), for systemic candidiasis rats were treated at 0, 4, 24, and 48 h after infection (lb.i.d.+2q.d.). Effective dose 50% (ED50) values were determined on day 14 after infection.

Table 3

(μmol/kg)

| | Systemic candidiasis | | Pulmonay aspergillosis | | Systemic aspergillosis | |
|---|---|---|---|---|---|---|
| | i.v. | p.o. | i.v. | p.o. | i.v. | p.o. |
| Example 16 | 3.5 | <2.1 | 17 | 18 | >35 | >55 |
| Example 23 | 4.6 | 4.7 | 8.0 | 17 | 17 | 19 |
| Itraconazole | n.t. | n.t. | n.t. | n.t. | n.t. | 17 |
| Fluconazole | n.t. | >2.9 | n.t. | n.t. | n.t. | n.t. |

Therefore, the water soluble azole antifungal agents, represented by the general formula (I) as well as salts, hydrates or solvates thereof, according to the present invention, exhibit potent antifungal activity against various fungal infections including Aspergillosis in mice over a very wide range of dosages both orally and parenterally and are useful as antifungal agents.

The present invention further relates to the pharmaceutical compositions containing the azole compound of the general formula (I) as well as salts, hydrates or solvates thereof.

The azole compounds of the formula (I) as well as salts, hydrates or solvates thereof are very active antimycotic agents. They are active against a variety of fungal species including *Candida spp.*, *Cryptotoccus neoformans*, *Aspergillus spp.*, *Trichophyton* spp., *Microsporum* spp., *Exophiala* spp., *Blastomyces dermatitidis*, and *Histoplasma capsulatum*.

Thus, the compounds of the present invention are useful for topical and systemic treatment of mycoses in animals as well as in humans. For example, they are useful in treating topical and mucosal fungal infections caused by, among other genera, *Candida*, *Trichophyton*, or *Microsporum*. They may also be used in the treatment of systemic fungal infections caused by, for example, *Candida spp.*, *Cryptococcus neoformans*, *Aspergillus spp.*, *Paracoccidiodes spp.*, *Sporotrix spp.*, *Exophiala spp.*, *Blastomyces spp.*, or *Histoplasma spp.*.

For clinical use, the azole compound of the formula (I) as well as salts, hydrates or solvates thereof can be administered alone, but will generally be administered in pharmaceutical admixture formulated as appropriate to the particular use and purpose desired, by mixing excipient, binding agent, lubricant, disintegrating agent, coating material, emulsifier, suspending agent, solvent, stabilizer, absorption enhancer and/or ointment base. The admixture can be used for oral, injectable, rectal or topical administration.

Pharmaceutical formulation for oral administration may be granule, tablet, sugar coated tablet, capsule, pill, suspension or emulsion. For parenteral injection, for example, intravenously, intramuscularly or subcutaneously, the azoles of formula (I) may be used in the form of a sterile aqueous solution which may contain other substances, for example, salts or glucose to make the solution isotonic. The boles can also be administered in the form of a suppository or pessary, or they may be applied topically in the form of a lotion, solution, cream, ointment or dusting powder.

The daily dosage level of the azole compounds of the formula (I) is from about 0.1 to about 50 mg/kg (in divided doses) when administered in one, two or more dosages by either the oral or parenteral route. Thus tablets or capsules of the compounds may contain from about 5 mg to about 0.5 g of active compound for administration. In any event the actual dosage can be determined by the physician and it may be varied upon the age, weight and response of the particular patient.

In addition, the azole compounds of the formula (I) as well as salts, hydrates or solvates thereof have activity against a variety of plant pathogenic fungi, including for example *Pyricularia oryzae*, *Pythium aphanidermatum*, *Alternaria* spp., and *Paecilomyces variotii*.

Thus, they can be applied for agricultural and horticultural purposes preferably in the form of a composition formulated as appropriate to the particular use and purpose desired, for example dusting powders, or granules, seed dressings, aqueous solutions, dispersions or emulsions, dips, sprays or aerosols. Such compositions may contain such conventional carriers, diluents or adjuvants as are known and acceptable in agriculture and horticulture. Other compounds having herbicidal or insecticidal, or additional antifungals can be incorporated in the compositions. The compounds and compositions can be applied in a number of ways, for example they can be applied directly to the plant foliage, stems, branches, seeds or roots or to the soil or other growing medium, and they may be used not only to eradicate the disease, but also prophylactically to protect the plants or seeds from attack.

The following examples illustrate the preferred methods for the preparation of the compounds of the present invention, which are not intended to limit the scope of the invention thereto.

Example 1

Preparation of 4-(4-Acetoxy-3,5-dimethylbenzyl)-1-[4-(4-{4-(4-(1-(2-butyl)-5-oxo-1,5-dihydro-[1,2,4]triazol-4-yl)phenyl]piperazin-1-yl}phenoxyethyl)-2-(2,4-dichlorophenyl)-[1,3]dioxolan-2-ylmethyl]-1H-[1,2,4]triazol-4-ium bromide

To a solution of 1g of 3,5-dimethyl-4-hydroxybenzyl bromide in $CHCl_3$-$CH_3CN$ (7/3ml) was added 400mg of Itraconazole and stirring was continued for 15h at room temperature. The solvent was evaporated in vacuo and the residue was stirred in acetic anhydride-pyridine (4/4ml) for 2h at ambient temperature. The mixture was concentrated and column chromatography on silica gel (200Å solvent: $CH_2Cl_2$/MeOH =10/1) gave 4-(4-acetoxy-3,5-dimethylbenzyl)-1-[4-(4-{4-[4-(1-(2-butyl)-5-oxo-1,5-dihydro-[1,2,4]triazol-4-yl)phenyl]piperazin-1-yl}phenoxymethyl)-2-(2,4-dichlorophenyl)-[1,3] dioxolan-2-ylmethyl]-1H-[1,2,4]triazol-4-iumbromide (507mg, 93%, as amorphous powder) ;

FAB-MS : m/z 881 (M-Br)+; 1H-NMR(CDCl3) d 0.90(3H,t,J=7.3Hz), 1.39(3H,d,J=6.9Hz), 1.69~1.77(1H,m), 1.81~1.87(1H,m), 2.02(3H,s), 2.10(3H,s), 2.32(3H,s), 3.22~3.29(2H,m), 3.31~3.38(1H,m), 3.66~3.74 (1H,m), 3.85~3.91(1H,m), 4.11~4.15(1H,m), 4.28~4.33(2H,m), 4.35 ~4.45(1H,m), 5.02(1H,d,J=14.2Hz), 5.03(1H,d,J=14.5Hz), 5.13(1H,d,J=14.5Hz), 5.54(1H,d,J=14.2Hz), 6.88~7.04(8H,m), 7.29(1H,dd,J=2.0,7.3Hz), 7.45(1H,d,J=8.9), 7.47(1H,d,J=2.0), 7.62(1H,s), 7.68(1H,d, J=8.2), 8.02(1H,s), 11.5~11.6(1H,brs).

The following compounds in Examples 2 ~ 8 were obtained according to a manner analogous to that of Example 1.

Example 2

4-(4-(Pyridine-3-carbonyloxy)-3,5-dimethylbenzyl)-1-[4-(4-{4-(4-(1-(2-butyl)-5-oxo-1,5-dihydro-[1,2,4]triazol-4-yl)phenyl]piperazin-1-yl}phenoxymethyl)-2-(2,4-dichlorophenyl)-[1,3]dioxolan-2-ylmethyl]-1H-[1,2,4]triazol-4-ium bromide;

Physical form : amorphous powder ; MALDI-TOF-MS : m/z 945 (M-Br)+ ; 1H-NMR(CD3OD) d 0.88(3H,t,J=7.6Hz), 1.36(3H,d,J=5.3Hz), 1.73(2H,m), 2.16(6H,s), 3.30(8H,m), 3.63~4.44(6H,m), 5.18(2H,s), 5.48(2H,s), 6.84~8.56(18H,m), 9.01(1H,s).

Example 3

4-(4-Benzoyloxy-3,5-dimethylbenzyl)-1-[4-(4-{4-[4-(1-(2-butyl)-5-oxo-1,5-dihydro-[1,2,4]triazol-4-yl)phenyl]piperazin-1-yl}phenoxym ethyl)-2-(2,4-dichlorophenyl)-[1,3]dioxolan-2-ylmethyl]-1H-[1,2,4]triazol-4-ium bromide;

Physical form : amorphous powder; FAB-MS : m/z 943 (M-Br)+; 1H-NMR(d6-DMSO) d 0.80(3H,t,J=7.3Hz), 1.29(3H,d,J=6.6Hz), 1.67(2H, m), 2.11(6H,s), 3.40(8H,brs), 3.77(2H,m), 3.95(2H,m), 4.12(1H,m), 4.40(1H,m), 5.08(2H,s), 5.42(2H,s), 6.90(2H,brd), 7.11~7.80(14H,m), 8.17(2H,d,J=7.2Hz), 8.35(1H,s), 9.40(1H,s), 10.4(1H,s).

Example 4

1-(4-Acetoxy-3,5-dimethyl-benxyl)-3-[(2R*,4S*)-4-[4-(4-acetyl-pipera zin-1-yl)-phenoxymethyl]-2-(2,4-dichloro-phenyl)-[1,3]dioxolan-2-ylmethyl]-3H-imidazol-1-ium bromide;

Physical form : pale brown oil ; FAB-MS : m/z 707 (M-Br)+ ; 1H-NMR(CDCl3) d 2.11(6H,s), 2.14(3H,s), 2.33(3H,s), 3.07(4H,m), 3.62(2H,m), 3.68(1H, dd,J=4.6,10.2Hz), 3.77(2H,m), 3.89~3.95(2H,m), 4.02(1H,dd,J=4.0,11.6Hz), 4.37(1H,m), 4.85(2H,s), 5.08(1H,d,J=14.2Hz), 5.48(1H,d,J=14.2Hz), 6.81(2H,d,J=8.9Hz), 6.91(2H,d,J=8.9Hz), 6.99(1H,s), 7.05(2H,s), 7.18(1H,s), 7.31(1H,dd,J=2.0,8.6Hz), 7.47(1H,d,J=2.0Hz), 7.69(1H,d,J=8.6Hz), 10.42(1H,s).

Example 5

3-[(2R*,4S*)-4-[4-(4-Acetyl-piperazin-1-yl)-phenoxymethyl]-2-(2,4-dichloro-phenyl)-[1,3]dioxolan-2-ylmethyl]-1-(4-isobutyryloxy-3,5-dimethyl-benzyl)-3H-imidazol-1-ium bromide;

Physical form : clear film ; FAB-MS: m/z 735 (M-Br)+ ; 1H-NMR(CDCl3) d 1.34(6H,d,J=6.9Hz), 2.09(6H,s), 2.14(3H,s), 2.85(1H, sept,J=6.9Hz), 3.06(4H,m), 3.61(1H,dd,J=4.9,5.2Hz), 3.67(1H,dd,J=4.0, 10.6Hz), 3.97(1H,dd,J=4.9,5.2Hz), 3.90(2H,m), 3.97(1H,dd,J=4.0,10.6Hz), 4.35(1H,m), 4.83(2H,s), 5.15(1H,d,J=14.5Hz), 5.49(1H,d,J=14.5Hz), 6.80(2H,d,J=8.9Hz), 6.91(2H,d,J=8.9Hz), 7.08(2H,s), 7.10(1H,d,J=2.0Hz), 7.19(1H,brs), 7.30(1H,dd,J=2.0,8.3Hz), 7.46(1H,d,J=2.0Hz), 7.67(1H,d,J=8.3Hz), 10.32(1H,brs).

Example 6

1-(4-Acetoxy-3,5-dichloro-benzyl)-3-[(2R*,4S*)-[4-[4-(4-acetyl-piperazin-1-yl)-phenoxymethyl]-2-(2,4-dichloro-phenyl)-[1,3]dioxolan-2-ylmethyl]-3H-imidazol-1-ium bromide;

Physical form : pale brown oil ; FAB-MS : m/z 747 (M-Br)+ ; 1H-NMR(CDCl3) d 2.14(3H,s), 2.39(3H,s), 3.07(4H,m), 3.62(3H,m), 3.78(2H,m), 3.89(1H,m), 3.97(1H,m), 4.10(1H,m), 4.39(1H,m), 4.75 (1H,d,J=16.2Hz), 4.83(1H,d,J=16.2Hz), 5.11(1H,d,J=14.8Hz), 5.77(1H,d,J=14.8Hz), 6.79(2H,d,J=9.1Hz), 6.92(2H,d,J=9.1Hz), 7.01(1H,s), 7.22(1H,s), 7.33(1H,dd,J=8.6,2.0Hz), 7.42(2H,s), 7.48(1H,d,J=2.0Hz), 7.68(1H,d,J=8.6Hz), 10.52(1H,s).

Example 7

dl-1-(4-Acetoxy-3,5-dimethyl-benzyl)-3-[2-(2,4-dichloro-benzyloxy)-2-(2,4-dichloro-phenyl)-ethyl]-3H-imidazol-1-ium bromide;

Physical form : amorphous powder ; FAB-MS : m/z 593 (M-Br)+ ; 1H-NMR(CD3OD) d 2.15(6H,s), 2.35(3H,s), 4.41~4.58(4H,m), 5.22(1H,dd,J=3.5,7.8Hz), 5.32(2H,s), 7.16(2H,brs), 7.24~7.56(7H,m), 7.66(1H,d,J=1.0Hz), 9.00(1H, brs).

Example 8

4-(4-acetoxy-3,5-dimethyl-benzyl)-1-[(1R,2S,6R)-2-methoxy-3,3-dimethyl-6-(2-*p*-tolyl-ethyl)-cyclohexylmethyl]-1H-[1,2,4]triazol-4-ium bromide ;

Physical form : colorless oil ; MALDI-TOF-MS : m/z 518 (M-Br)+ ; 1H-NMR(CDCl3) d 0.84(3H,s), 1.02(3H,s), 1.10~1.30(3H,m), 1.37~1.42 (1H,m), 1.45~1.65(1H,m), 1.71(1H,m), 1.93(2H,m), 2.15(6H,s), 2.31 (3H,s), 2.34(3H,s), 2.51(1H,m), 2.71(1H,m), 2.83(1H,d,J=10.9Hz), 3.50 (3H,s), 4.49(1H,d,J=18.9Hz), 4.60(1H,dd,J=18.9,5.9Hz), 5.58(1H,d,J=14.4Hz), 5.66(1H,d,J=14.4Hz), 7.08(4H,s), 7.21(2H,s), 8.31(1H,s), 11.64(1H,s).

Example 9

Preparation of 1-(4-Acetoxybenzyl)-3-[(2R*,4S*)-4-[4-(4-acetylpiper azin-1-yl)phenoxymethyl]-2-(2,4-dichlorophenyl)-[1,3]dioxolan-2-yl methyl]-3H-imidazol-1-ium bromide

To a solution of 28mg of 4-acetoxybenzyl bromide in 1.5mL of CHCl3 was added 30mg of Ketoconazole and the mixture was stirred for 16h at room temperature. The solvent was evaporated in vacuo. Column chromatography on silica gel(Wakogel C-200, solvent:CH2Cl2 /MeOH=10/1) gave 1-(4-acetoxybenzyl)-3-[(2R*,4S*)-4-[4-(4-acetyl pi perazin-1-yl)phenoxymethyl]2-(2,4-dichlorophenyl)-[1,3]dioxolan-2 -yl methyl]-3H-imidazol-1-ium bromide (32mg , 76% , as colorless oil); MALDI-TOF-MS : m/z 679 (M-Br)+; 1H-NMR(CDCl3) d 2.14(3H,s), 2.28(3H,s), 3.05(4H,m), 3.60~3.89(8H,m), 4.33(1H,m), 4.80(2H,s), 5.37(1H,d,J=14.5Hz), 5.61(1H,d,J=14.5Hz), 6.78(2H,d,J=9.1Hz), 6.92, (2H,d,J=9.1Hz), 7.03(2H,d,J=8.6Hz), 7.24~7.63(7H,m), 10.1(1H,s).

The following compounds in Example 10~13 were obtained according to a manner analogous to that of Example 9.

Example 10

3-[(2R*,4S*)-4-[4-(4-Acetylpiperazin-1-yl)phenoxymethyl]-2-(2,4-dichlorophenyl)-[1,3]dioxolan-2-ylmethyl]-1-[4-(2,2-dimethylpropionyloxy)benzyl]-3H-imidazol-1-ium bromides;

Physical form : amorphous powder ; MALDI-TOF-MS : m/z 721 (M-Br)+ ; 1H-NMR (CDCl3) d 1.34(9H,s), 2.14(3H,s), 3.06(4H,m), 3.60-4.00(8H,m),4.36, (1H,m), 4.81(2H,s), 5.29(1H,d,J=14.4Hz), 5.62(1H,d,J=14.4Hz), 6.80(2H,d,J=9.3Hz), 6.92(2H,d,J=9.3Hz), 7.02(2H,d,J=8.6Hz), 7.15(1H,brs), 7.20(1H,brs), 7.28(1H,brs), 7.43(2H,d,J=8.6Hz), 7.46(1H,brs), 7.65(1H,d,J=8.3Hz), 10.3(1H,s).

Example 11

1-(4-Acetoxy-3-methyl-benzyl)-3-[(2R*,4S*)-4-[4-(4-acetyl-piperazin-1-yl)-phenoxymethyl]-2-(2,4-dichloro-phenyl)-[1,3]dioxolan-2-ylmethyl]-3H-imidazol-1-ium bromide ;

Physical form : clear film ; FAB-MS: m/z 693(M-Br)+; 1H-NMR(CDCl3) d 2.13(6H,s), 2.31(3H,s), 3.06(4H,dt,J=5.0Hz), 3.62(2H,t,J=5.0Hz), 3.66(1H,dd,J=4.6Hz), 3.76(2H,t,J=9.0Hz), 3.88(2H,m), 3.94(1H,dd,J=4.6Hz), 4.36(1H,m), 4.81(2H,s), 5.26(1H,d,J=14.0Hz), 5.55(1H,d,J=14.0Hz), 6.79(2H,d,J=9.0Hz), 6.91(2H,d,J=9.0Hz), 6.96 (1H,d,J=8.0Hz), 7.22(2H,brs), 7.23(1H,dd,J=2.0,8.0Hz), 7.29(1H,dd,J=2.0,8.0Hz), 7.39(1H,brd), 7.45(1H,d,J=2.0Hz), 7.64(1H,d,J=8.0Hz), 10.21(1H,s).

Example 12

1-(4-Acetoxy-3-methoxyl-benzyl)-3-[(2R*,4S*)-4-[4-(4-acetyl-piperazin-1-yl)-phenoxyethyl]-2-(2,4-dichloro-phenyl)-[1,3]dioxolan-2-yl methyl]-3H-imidazol-1-ium bromide ;

Physical form: amorphous powder ; MALDI-TOF-MS : m/z 709(M-Br)+; 1H-NMR(CDCl3) d 2.14(3H,s), 2.30(3H,s), 3.06(4H,m), 3.62(2H,m), 3.65(1H,dd,J=4.6,10.2Hz), 3.77(2H,m), 3.86(3H,s), 3.88~3.94(2H,m), 4.03(1H,dd,J=4.0,11.6Hz), 4.36(1H,m), 4.80(2H,s), 5.12(1H,d,J=14.2Hz), 5.54(1H,d,J=14.2Hz), 6.80(2H,d,J=8.9Hz), 6.81(1H,s), 6.92 (2H,d,J=8.9Hz), 6.95(1H,s), 7.07(1H,s), 7.17(1H,s), 7.31(1H,dd,J=2.0,8.6Hz), 7.44(1H,d,J=2.0Hz), 7.47(1H,J=2.0Hz), 7.66(1H,d,J=8.6Hz), 10.41(1H,s).

Example 13

dl-1-[4-(4-{4-[4-(1-sec-butyl-5-oxo-1,5-dihydro-[1,2,4]triazol-4-yl)-phenyl]-piperazin-1-yl}-phenoxymethyl)-2-(2,4-dichloro-phenyl)-[1,3]dioxolan-2-ylmethyl]-4-(4-hexanoyloxy-3,5-dimethyl-benzyl)-1H-[1,2,4]triazol-4-ium methanesulfonate ;

Physical form : amorphous powder ; FAB-MS : m/z 937(M-MsO)+; 1H-NMR(CDCl3) $\delta$ 0.80~1.00(6H,m), 1.42(3H,d,J=6.6Hz), 1.20~1.50 (6H,m), 1.70~2.00(2H,m), 2.10(9H,s), 2.58(2H,t,J=7.6Hz), 3.26(4H,m), 3.36(4H,m),3.62~3.70(2H,m), 3.75(1H,m), 4.10~4.50(3H,m), 4.84(1H,d,J=14.2Hz), 5.00(1H,d,J=13.8Hz), 5.12(1H,d,J=13.8Hz), 5.44(1H,d,J=14.2Hz), 6.90(2H,d,J=8.9Hz), 6.94(2H,s), 6.99(2H,d,J=8.9Hz), 7.03(2H,d,J=8.9Hz), 7.31(1H,dd,J=8.6,2.0Hz), 7.43(2H,d,J=8.9HZ), 7.48(1H,d,J=2.0Hz), 7.62(1H,s), 7.71(1H,d,J=8.6Hz), 7.90(1H,s), 11.30(1H,s).

Example 14

(2R,3R)-4-(4-Acetyloxy-3,5-dimethylbenzyl)-1-[3-[4-(4-cyanophenyl)-thiazol-2-yl]-2-(2,4-difluorophenyl)-2-hydroxy-butyl]-1H-[1,2,4]triazol-4-ium bromide;

Physical form : amorphous poweder ; FAB-MS : m/z 614 $(M-Br)^+$ ; $^1$H-NMR($CD_3OD$) d 1.24(3H,d,J=6.9Hz), 2.25(6H,s), 2.36(3H,s), 4.30(1H,q,J=6.9Hz), 4.64(1H,d,J=14.2Hz), 5.10(1H,d,J=14.2Hz), 5.31(2H,s), 6.75-7.27(3H,m), 7.10(2H,s), 7.82(2H,d,J=8.3Hz), 8.09(1H,s), 8.18(2H,d,J=8.2Hz), 8.73(1H,s), 9.95(1H,s)

Example 15

(2R,3R)-4-(4-Aminoacetoxy-2-carboxy-benzyl)-1-[3-[4-(4-cyano-phenyl)-thiazol-2-yl]-2-(2,4-difluorophenyl)-2-hydroxy-butyl]-1H-[1,2,4]triazol-4-ium bromide trifluoroacetic acid salt

a) Preparation of 3-(tert-butoxycarbonylaminoacetoxy)-6-(bromomethyl)benzoic acid tert-butyl ester
A mixture of 200mg of 3-hydroxy-6-(hydroxymethyl)benzoic acid tert-butyl ester, 187mg of Boc-glycine, 65mg of 4-dimethylaminopyridine and 245mg of 1-(3-dimethylaminopropyl)-3-ethylcarbodiimide hydrochloride in 8mL of dichloromethane was stirred for 15h at room temperature and was diluted with 40mL of dichloromethane. The mixture was washed with 1N HCl and dried over anhydrous $Na_2SO_4$.
Removal of the solvent gave 180mg of 3-(tert-butoxycarbonylaminoacetoxy)-6-hydroxymethylbenzoic acid tert-butyl ester.
To a solution of 650mg of 3-(tert-butoxycarbonylaminoacetoxy)-6-hydroxymethylbenzoic acid tert-butyl ester and 580mg of triphenylphosphine was added 848mg of carbon tetrabromide, and the mixture was stirred for 2h at room temperature. The mixture was diluted with dichloromethane and washed with water. The organic layer was dried over anhydrous $Na_2SO_4$ and concentrated in vacuo. Column chromatography on silica gel gave 322mg of 3-(tert-butoxycarbonylaminoacetoxy)-6-(bromomethyl)benzoic acid tert-butyl ester as a colourless oil; $^1$H-NMR($CDCl_3$) d 1.34(9H,s), 1.51(9H,s), 4.08(2H,br.d,J=5.6Hz), 4.80(2H,s), 4.95(1H,br.s), 7.12(1H,dd,J=8.6,2.6Hz), 7.34(1H,d,J=8.6Hz), 7.51(1H,d,J=2.6Hz)

b) (2R,3R)-4-(4-Aminoacetoxy-2-carboxybenxyl)-1-[3-[4-(4-cyanophenyl)thiazol-2-yl]-2-(2,4-difluorophenyl)-2-hydroxybutyl]- 1H-[1,2,4]triazol-4-ium bromide trifluoroacetic acid salt was obtained as a white solid from (1R,2R)-4-[2-[2-(2,4-difluoro-phenyl)-2-hydroxy-1-methyl-3-[1,2,4]triazol-1-yl-propyl]-thiazol-4-yl]-benzonitrile and 3-(tert-butoxycarbonylaminoacetoxy)-6-(bromomethyl)benzoic acid tert-butyl ester according to a manner analogous to that of Example 1;

Physical form : white powder ; FAB-MS : m/z 645 $(M-Br)^+$ ; $^1$H-NMR($CD_3OD$) d 1.22(3H,d,J=7.3Hz), 4.21(2H,s), 4.28(1H,q,J=7.3Hz), 4.61(1H,d,J=14.2Hz), 5.09(1H,d,J=14.2Hz), 5.68(1H,d,J=14.0Hz), 5.75(1H,d,J=14.0Hz), 6.78-7.28(3H,m), 7.55(1H,dd,J=2.3,8.6Hz), 7.64(1H,d,J=8.6Hz), 7.81(2H,d,J=8.5Hz), 8.03(1H,d,J=2.3Hz), 8.10(1H,s), 8.17(2H,d,J=8.5Hz), 8.67(1H,s), 9.80(1H,s)

Example 16

1-[(2R,3R)-3-[4-(4-Cyanophenyl)-thiazol-2-yl]-2-(2,4-difluorophenyl)-2-hydroxybutyl]-4-[(S)-3,5-dimethyl-4-(pyrrolidine-2-carbonyloxy)-benzyl]-1H-[1,2,4]triazol-4-ium bromide trifluoroacetic acid salt
was obtained either by the following method A or B.

i) method A:
To a solution of 3g of 3,5-dimethyl-4-hydroxybenzyl bromide in $CH_3CN$(30mL) was added 1.2g of (1R,2R)-4-[2-[2-(2,4-difluorophenyl)-2-hydroxy-1-methyl-3-[1,2,4]triazol-1-yl-propyl]thiazol-4-yl]benzonitrile and stirring was continued for 2h at room temperature. The precipitate was filtered and washed with ether to give 1.37g(81%y.) of 1-[(2R,3R)-3-[4-(4-cyanophenyl)thiazol-2-yl]-2-(2,4-difluorophenyl)-2-hydroxybutyl]-4-(3,5-dimethyl-4-hydroxy)benzyl-1H-[1,2,4]triazol-4-ium bromide(Ro09-3846) as a white solid.
To a mixture of 30g of 1-[(2R,3R)-3-[4-(4-cyanophenyl)thiazol-2-yl]-2-(2,4-duluoro-phenyl)-2-hydroxybutyl]-4-(3,5-dimethyl-4-hydroxybenzyl)-1H-[1,2,4]triazol-4-ium bromide, 10.9g of Boc-(L)-proline and 2.8g of N,N-dimethylaminopyridine in 1.2L of dichloromethane was added 17.6g of 1-(3-dimethylaminopropyl)-3-ethylcarbodiimide hydrochloride and the mixture was stirred for 1.5h at room temperature and concentrated in vacuo. The residue was chromatographed on silica gel (Wakogel C-200, solvent : $CH_2Cl_2$ / MeOH=14 / 1) to give 30.9g(79%y.) of 1-[(2R,3R)-3-[4-(4-cyanophenyl)thiazol-2-yl]-2-(2,4-difluorophenyl)-2-hydroxybutyl]-4-[(S)-3,5-dimethyl-4-(N-tert-butoxycarbonylpyrrolidine-2-carbonyloxy)benzyl]-1H-[1,2,4]triazol-4-ium bromide as a white solid.
30.9g of 1-[(2R,3R)-3-[4-(4-cyanophenyl)thiazol-2-yl]-2-(2,4-difluorophenyl)-2-hydroxybutyl]-4-[(S)-3,5-dimethyl-4-(N-tert-butoxycarbonylpyrrolidine-2-carbonyloxy)benzyl]-1H-[1,2,4]triazol-4-ium bromide was stirred in 600mL of 10% TFA - dichloromethane for 5h at room temperature and the solvent was removed in vacuo. The residue was diluted with ether and the precipitate was washed with ether to give 1-[(2R,3R)-3-[4-(4-cyanophenyl)thiazol-2-yl]-2-(2,4-difluorophenyl)-2-hydroxybutyl]-4-[(S)-3,5-dimethyl-4-(pyrrolidine-2-carbonyloxy)benzyl]-1H-[1,2,4]triazol-4-ium bromide trifluoroacetic acid salt as a white solid.

ii) method B:

A mixture of 1.06g of (1R,2R)-4-[2-[2-(2,4-difluorophenyl)-2-hydroxy-1-methyl-3-[1,2,4]triazol-1-yl-propyl]thiazol-4-yl]benzonitrile and 1.1g of 3,5-dimethyl-4-[(S)- N-tert-butoxycarbonylpyrrolidine-2-carbonyloxy]benzyl bromide, prepared from 3,5-dimethyl-4-hydroxybenzaldehyde in 3 steps, in 20mL of acetonitrile was stirred for 15h at reflux temperature and then concentrated. The residue was chromatographed on silica gel (Wakogel C-200, solvent : $CH_2Cl_2$ / MeOH=12 / 1) to give 1.92g (94%y.) of 1-[(2R,3R)-3-[4-(4-cyanophenyl)thiazol-2-yl]-2-(2,4-difluorophenyl)-2-hydroxybutyl]-4-[(S)-3,5-dimethyl-4-(N-tert-butoxycarbonylpyrrolidine-2-carbonyloxy)benzyl]-1H-[1,2,4]triazol-4-ium bromide as a white solid.

1-[(2R,3R)-3-[4-(4-Cyanophenyl)thiazol-2-yl]-2-(2,4-difluorophenyl)-2-hydroxybutyl]-4-[(S)-3,5-dimethyl-4-(pyrrolidine-2-carbonyloxy)benzyl]-1H-[1,2,4]triazol-4-ium bromide trifluoroacetic acid salt was obtained by removal of the protecting group as described in the method A;

Physical form : white powder ; MALDI-TOF-MS : m/z 669 $(M-Br)^+$; $^1$H-NMR($CD_3OD$) d 1.24(3H,d,J=7.3Hz), 2.18-2.25(2H,m), 2.21(6H,s), 2.36-2.44(1H,m), 2.66-2.71(1H,m), 3.43-3.50(2H,m), 4.30(1H,q,J=7.3Hz), 4.65(1H,d,J=14.2Hz), 4.88(1H,m), 5.12(1H,d,J=14.2Hz), 5.33(2H,s), 6.73-6.79(1H,m), 6.99-7.06(1H,m), 7.14(2H,s), 7.21-7.30(1H,m), 7.82(2H,d,J=8.6Hz), 8.10(1H,s), 8.18(2H,d,J=8.6Hz), 8.74(1H,s), 10.00(1H,s)

Following compounds in Example 17-30 were obtained according to a manner analogous to that of Example 16.

Example 17

(2R,3R)-4-(4-Aminoacetoxy-3,5-dimethylbenzyl)-1-[3-[4-(4-cyanophenyl)-thiazol-2-yl]-2-(2,4-difluorophenyl)-2-hydroxybutyl]-1H-[1,2,4]triazol-4-ium bromide trifluoroacetic acid salt;

Physical form : white powder ; FAB-MS : m/z 629 $(M-Br)^+$ ; $^1$H-NMR($CD_3OD$) d 1.25(3H,d,J=7.3Hz), 2.21(6H,s), 4.29(1H,q,J=7.3Hz), 4.31(2H,s), 4.66(1H,d,J=14.2Hz), 5.12(1H,d,J=14.2Hz), 5.34(2H,s), 6.72-7.30(3H,m), 7.15(2H,s), 7.81(2H,d,J=6.6Hz), 8.10(1H,s), 8.17(2H,d,J=6.6Hz), 8.74(1H,s), 10.0(1H,s)

Example 18

(2R,3R)-4-[4-(3-Aminopropionyloxy)-3,5-dimethylbenzyl]-1-[4-[4-(4-cyanophenyl)-thiazol-2-yl]-2-(2,4-difluorophenyl)-2-hydroxy-3-methylbutyl]-1H-[1,2,4]triazol-4-ium bromide trifluoroacetic acid salt;

Physical form : white powder ; FAB-MS : m/z 643 $(M-TFA-Br)^+$; $^1$H-NMR($CD_3OD$) d 1.24(3H, t, J=7.3Hz), 2.19(6H, s), 3.09~3.37(4H, m), 4.30(1H, q, J=7.3Hz), 4.66(1H, d, J=14.2Hz), 5.11(1H, d, J=14.2Hz), 5.33(2H, br. s), 6.73~6.81(1H, m), 6.98-7.07(1H, m), 7.12(2H, s), 7.20-7.33(1H, m), 7.81(2H, d, J=6.9Hz), 8.10(1H, s), 8.18(2H, d, J=6.9Hz), 8.74(1H, s), 10.00(1H, s).

Example 19

(2R,3R)-4-[4-(4-Aminobutyryloxy)-3,5-dimethylbenzyl]-1-[4-[4-(4-cyanophenyl)-thiazol-2-yl]-2-(2,4-difluorophenyl)-2-hydroxy-3-methylbutyl]-1H-[1,2,4]triazol-4-ium bromide trifluoroacetic acid salt;

Physical form : white powder; FAB-MS : m/z 657 $(M-TFA-Br)^+$ ; $^1$H-NMR($CD_3OD$) d 1.24(3H, d, J=7.26Hz), 2.03~2.12(2H, m), 2.17(6H, s), 2.87(2H, t, J=7.5Hz), 3.07(2H, t, J=7.5Hz), 4.30(1H, t, J=7.5Hz), 4.65(1H, d, J=14.4Hz) 5.10(1H, d, J=14.4Hz), 5.31(2H, s),
6.73~6.80(1H, m), 6.98-7.07(1H, m), 7.11(2H, s), 7.18-7.28(1H, m), 7.82(2H, d, J=8.6Hz), 8.10(1H, s), 8.17(2H, d, J=8.6Hz), 8.73(1H,s).

Example 20

(2R,3R)-4-[4-[(2-Aminoacetylamino)acetoxy]-3,5-dimethylbenzyl]-1-[3-[4-(4-cyanophenyl)-thiazol-2-yl]-2-(2,4-difluorophenyl)-2-hydroxybutyl]-1H-[1,2,4]triazol-4-ium bromide trifluoroacetic acid salt;

Physical form : white powder; FAB-MS : m/z 686 $(M-Br)^+$ ; $^1$H-NMR(DMSO-$d_6$) d 1.18(3H,d,J=6.9Hz), 2.08(6H,s), 3.67(2H,brs), 4.14(1H,q,J=6.9Hz), 4.34(2H,d,J=5.6), 4.69(1H,d,J=14.2Hz), 5.01(1H,d,J=14.2Hz), 5.35(2H,s), 6.58(1H,s), 6.90-6.96(1H,m), 7.07(2H,s), 7.21-7.37(2H,m), 7.94(2H,d,J=7.9Hz), 8.07(2H,brs), 8.21(2H,d,J=7.9Hz), 8.43(1H,s), 8.99(1H,t,J=5.6), 9.02(1H,s), 10.06(1H,s).

Example 21

1-[(2R,3R)-3-[4-(4-Cyanophenyl)-thiazol-2-yl]-2-(2,4-difluorophenyl)-2-hydroxy-butyl]-4-[4-[(S)-2,5-diaminopentoyloxy]-3,5-dimethylbenzyl]-1H-[1,2,4]triazol-4-ium bromide trifluoroacetic acid salt;

Physical form : white powder ; FAB-MS : m/z 686 $(M-Br)^+$; $^1$H-NMR($CD_3OD$) d 1.24(3H,d,J=6.9Hz), 1.95-2.43(4H,m), 2.22(6H,s), 3.09(2H,t,J=7.0Hz), 4.30(1H,q,J=6.9Hz), 4.60(1H,m), 4.67(1H,d,J=14.2Hz), 5.12(1H,d,J=14.2Hz), 5.34(2H,s), 6.76-7.27(3H,m), 7.15(2H,s), 7.82(2H,d,J=8.2Hz:), 8.10(1H,s), 8.18(2H,d,J=8.2Hz), 8.74(1H,s), 10.0(1H,s)

Example 22

4-[4-[(S)-2-Aminopropionyloxy)-3,5-dimethylbenzyl]-1-[(2R,3R)-3-[4-(4-cyanophenyl)-thiazol-2-yl]-2-(2,4-difluorophenyl)-2-hydroxybutyl]-1H-[1,2,4]triazol-4-ium bromide trifluoroacetic acid salt;

Physical form : white powder; FAB-MS : m/z 643 $(M-Br)^+$; $^1$H-NMR($CD_3OD$) d 1.23(3H,d,J=6.9Hz), 1.79(3H,d,J=6.9Hz), 2.20(6H,s), 4.29 (1H,q,J=6.9Hz), 4.57(1H,q,J=6.9Hz), 4.67(1H,d,J=14.2Hz), 5.12(1H,d,J=14.2Hz), 5.34(2H,s), 6.70-7.26(3H,m), 7.14(2H,s), 7.80(2H,d,J=8.2Hz), 8.10(1H,s), 8.18(2H,d,J=8.2Hz), 8.74(1H,s), 10.0(1H,s)

Example 23

1-[(2R,3R)-3-[4-(4-Cyanophenyl)thiazol-2-yl]-2-(2,4-difluorophenyl)-2-hydroxybutyl]-4-[3,5-dimethyl-4-[(methylamino)acetoxy]benzyl]-1H-[1,2,4]triazol-4-ium bromide trifluoroacetic acid salt;

Physical form : amorphous powder ; FAB-MS : m/z 643 $(M-Br)^+$ ; $^1$H-NMR($CD_3OD$) d 1.24(3H,d,J=7.3Hz), 2.21(6H,s), 2.84(3H,s), 4.30(1H,q,J=7.3Hz), 4.45(2H,s), 4.65(1H,d,J=14.2Hz), 5.11(1H,d,J=14.2Hz), 5.33(2H,s), 6.76(1H,m), 7.02(1H,m), 7.14(2H,s), 7.24(1H,m), 7.82(2H,d,J=8.6Hz), 8.10(1H,s), 8.18(2H,d,J=8.6Hz), 8.74(1H,s), 9.99(1H,s).

Example 24

(2R,3R)-1-[3-[4-(4-Cyanophenyl)thiazol-2-yl]-2-(2,4-difluorophenyl)-2-hydroxybutyl]-4-[3,5-dimethyl-4-[(propylamino)acetoxy]benzyl]-1H-[1,2,4]triazol-4-ium bromide trifluoroacetic acid salt;

Physical form : white powder ; FAB-MS : m/z 671 $(M-Br)^+$ ; $^1$H-NMR($CD_3OD$) d 1.05(3H,t,J=7.4Hz), 1.25(3H,d,J=7.3Hz), 1.77(2H,m), 2.21(6H,s), 3.10(2H,m), 4.31(1H,q,J=7.4Hz), 4.47(2H,s), 4.66(1H,d,J=13.9Hz), 5.12(1H,d,J=13.9Hz), 5.33(2H,s), 6.72-7.29(3H,m), 7.14(2H,s), 7.82(2H,d,J=8.3Hz), 8.09(1H,s), 8.18(2H,d,J=8.3Hz), 8.74(1H,s), 10.0(1H,s)

Example 25

1-[(2R,3R)-3-[4-(4-Cyanophenyl)thiazol-2-yl]-2-(2,4-difluorophenyl)-2-hydroxybutyl]-4-[4-[(S)-2-(methylamino)propionyloxy]-3,5-dimethylbenzyl]-1H-[1,2,4]triazol-4-ium bromide trifluoroacetic acid salt;

Physical form : white powder ; FAB-MS : m/z 657 $(M-Br)^+$; $^1$H-NMR($CD_3OD$) d 1.25(3H,d,J=7.3Hz), 1.83(3H,d,J=7.3Hz), 2.21(6H,s), 2.83(3H,s), 4.26(1H,q,J=7.3Hz), 4.57(1H,q,J=7.3Hz), 4.66(1H,d,J=14.5Hz), 5.12(1H,d,J=14.5Hz), 5.34(2H,s), 6.72-7.26(3H,m), 7.15(2H,s), 7.82(2H,d,J=8.6Hz), 8.11(1H,s), 8.18(2H,d,J=8.6Hz), 8.75(1H,s)

Example 26

(2R,3R)-1-[4-[4-(4-cyanophenyl)thiazol-2-yl]-2-(2,4-difluorophenyl)-2-hydroxy-3-methylbutyl]-4-[3,5-dimethyl-4-[3-(methylamino)propionyloxy]benzyl]-1H-[1,2,4]triazol-4-ium bromide trifluoroacetate;

Physical form : white powder; FAB-MS: m/z 657 $(M-TFA-Br)^+$ ; $^1$H-NMR($CD_3OD$) d 1.24(3H, d, J=6.9Hz), 2.19(6H, s), 2.78(3H, s), 3.21(2H, t, J=6.6Hz), 3.42(2H, t, J=6.6Hz), 4.30(1H, q, J=6.9Hz), 4.66(1H, d, J=14.2Hz), 5.11(1H, d, J=14.2Hz), 5.33(2H, s), 6.73~6.81(1H, m), 6.98~7.07(1H, m), 7.12(2H, s), 7.20-7.30(1H, m), 7.82(2H, d,

J=8.7Hz), 8.10(1H, s), 8.18(2H, d, J=8.7Hz), 8.73(1H,s), 9.98(1H, s)

Example 27

4-[4-[(S)-2-Amino-3-methylbutanoyloxy]-3,5-dimethylbenzyl]-1-[(2R,3R)-3-[4-(4-cyanophenyl)thiazol-2-yl]-2-(2,4-difluoro-phenyl)-2-hydroxybutyl]-1H-[1,2,4]triazol-4-ium bromide trifluoroacetic acid salt;

Physical form : white powder; FAB-MS : m/z 671 $(M-Br)^+$; $^1$H-NMR($CD_3OD$) d 1.21(3H,d,J=6.9Hz), 1.25(3H,d,J=6.9Hz), 1.27(3H,d,J=6.9Hz), 2.22(6H,s), 2.66(1H,m), 4.27(1H,q,J=6.9Hz), 4.49(1H,d,J=3.6Hz), 4.66(1H,d,J=14.2Hz), 5.12(1H,d,J=14.2Hz), 5.35(2H,s), 6.73-7.30(3H,m), 7.15(2H,s), 7.82(2H,d,J=8.6Hz), 8.10(1H,s), 8.18(2H,d,J=8.6Hz), 8.75(1H,s), 10.0(1H,s)

Example 28

(2R,3R)-1-[3-[4-(4-Cyanophenyl)-thiazol-2-yl]-2-(2,4-difluorophenyl)-2-hydroxy-butyl]-4-[4-[(isopropylamino)acetoxy]-3,5-dimethylbenzyl]-1H-[1,2,4]triazol-4-ium bromide trifluoroacetic acid salt;

Physical form : amorphous powder ; FAB-MS : m/z 671 $(M-Br)^+$; $^1$H-NMR($CD_3OD$) d 1.24(3H,d,J=6.9Hz), 1.41(6H,d,J=6.6Hz), 2.22(6H,s), 3.32-3.53(1H,m), 4.30(1H,q,J=7.3Hz), 4.50(2H,s), 4.66(1H,d,J=14.2Hz), 5.12(1H,d,J=14.2Hz), 5.34(2H,s), 6.73-6.78(1H,m), 6.98-7.06(1H,m), 7.15(2H,s), 7.20~7.29(1H,m), 7.82(2H,d,J=8.6Hz), 8.11(1H,s), 8.18(2H,d,J=8.6Hz), 8.74(1H,s).

Example 29

1-[(2R,3R)-3-[4-(4-Cyanophenyl)thiazol-2-yl]-2-(2,4-difluorophenyl)-2-hydroxybutyl]-4-[(2S)-4-[2-(ethylamino)propionyloxy]-3,5-dimethylbenzyl]-1H-[1,2,4]triazol-4-ium bromide trifluoroacetic acid salt;

Physical form : amorphous powder ; MALDI-TOF-MS : m/z 671 $(M-Br)^+$ ; $^1$H-NMR($CD_3OD$) d 1.24(3H,d,J=7.3Hz), 1.38(3H,t,J=7.3Hz), 1.83(3H,d,J=7.3Hz), 2.21(6H,s), 3.14~3.25(2H,m), 4.30(1H,q,J=7.3Hz), 4.63(1H,q,J=7.3Hz), 4.66(1H,d,J=14.2Hz), 5.12(1H,d,J=14.2Hz), 5.35(2H,s), 6.74~6.80(1H,m), 6.98-7.07(1H,m), 7.16(2H,s), 7.21-7.30(1H,m), 7.82(2H,d,J=8.6Hz), 8.11(1H,s), 8.18(2H,d,J=8.6Hz), 8.75(1H,s), 10.00(1H,s).

Example 30

(2R,3R)-1-[3-[4-(4-Cyanophenyl)-thiazol-2-yl]-2-(2,4-difluorophenyl)-2-hydroxybutyl]-4-[4-[(ethylamino)acetoxy]-3,5-dimethylbenzyl]-1H-[1,2,4]triazol-4-ium bromide trifluoroacetic acid salt;

Physical form white powder; FAB-MS : m/z 657 $(M-Br)^+$ ; $^1$H-NMR($CD_3OD$) d 1.25(3H,d,J=7.3Hz), 1.38(3H,t,J=7.3Hz), 2.22(6H,s), 3.22(2H,q,J=7.3Hz), 4.27(1H,q,J=7.3Hz), 4.48(2H,s), 4.67(1H,d,J=14.2Hz), 5.12(1H,d,J=14.2Hz), 5.35(2H,s), 6.73-7.29(3H,m), 7.15(2H,s), 7.82(2H,d,J=8.6Hz), 8.10(1H,s), 8.18(2H,d,J=8.6Hz), 8.75(1H,s), 10.0(1H,s)

Example 31

1-[2R,3R)-3-[4-(4-Cyano-phenyl)-thiazol-2-yl]-2-(2,4-difluorophenyl)-2-hydroxy-butyl]-4-(3,5-dimethyl-4-methylaminoacetoxy-benzyl)-1H-[1,2,4]triazol-4-ium bromide hydrochloric acid salt

4-[4[(tert-Butoxycarbonyl-methyl-amino)acetoxy]-3,5-dimethyl-benzyl]-1-[2R,3R)-3-[4-(4-cyano-phenyl)-thiazole-2-yl]-2-(2,4-difluoro-phenyl)-2-hydroxy-butyl]-1H-[1,2,4]triazole-4-ium bromide (1:1) was obtained by the similar procedure as described in the method B. To a solution of 4-{4-[(tert-butoxycarbonyl-methyl-amino)-acetoxy]-3,5-dimethyl-benzyl}-1-[2R,3R)-3-[4-(4-cyano-phenyl)-thiazole-2-yl]-2-(2,4-difluoro-phenyl)-2-hydroxy-butyl]-1H-[1,2,4]triazole-4-ium bromide (1:1) (198.7 g, 241 mmol) in EtOAc (1000 mL), a solution of HCl in EtOAc (4 N, 600 mL) was added at room temperature over a period of 30 min with vigorous stirring. The resulting suspension was stirred at room temperature for 3 h. Precipitates were collected on a glass filter (3G) and washed with ether (500 mL x 10) and dried under vacuum at 40°C for 2 day and then at 80°C for 12 h to yield 1-[2R,3R)-3-[4-(4-cyano-phenyl)-thiazol-2-yl]-2-(2,4-difluoro-phenyl)-2-hydroxy-butyl]-4-(3,5-dimethyl-4-methylaminoacetoxy-benzyl)-1H-[1,2,4]triazol-4-ium bromide hydrochloric acid salt(183.3 g, 100%) as a colorless powder.

Physical form : amorphous powder ; FAB-MS : m/z 643 $(M-Br)^+$ ; 1H-NMR(DMSO-$d_6$) $\delta$ 1.18 (3H,d,J=7.3Hz), 2.12(6H,s), 2.64(3H,s), 4.13(1H,q,J=7.3), 4.42(2H,s), 4.72(1H,d,J=14.2), 5.01(1H,d,J=14.2), 5.39(2H,s), 6.70(1H,s), 6.90-6.94(1H,m), 7.13(2H,m), 7.26-7.34(2H,m), 7.94(2H,d,J=8.3), 8.20(2H,d,J=8.3), 8.44(1H,s), 9.07(1H,s), 9.51(2H,brs),. 10.17(1H,s)

Example 32

The following compounds can be prepared according to a manner similar to Example 1 or 9:

4-(4-Acetoxy-3,5-dimethylbenzyl)-1-[(2R,3R)-2-(2,4-difluorophenyl)-2-hydroxy-3-{5-oxo-4-[4-(2,2,3,3-tetrafluoropropoxy)phenyl]-4,5-dihydro-[1,2,4]triazol-1-yl}butyl]-1H-[1,2,4]triazol-4-ium bromide,
4-(4-acetoxy-3,5-dimethylbenzyl)-1-[(2R)-2-(2,4-difluorophenyl)-2-hydroxy-3-methyl-3-(6-[1,2,4]triazol-1-yl-pyridazin-3-ylsulfanyl)butyl]-1H-[1,2,4]triazol-4-ium bromide,
4-(4-acetoxy-3,5-dimethylbenzyl)-1-[(2R)-2-(2,4-difluorophenyl)-2-hydroxy-3-(3-{(Z)-2-[4-(2,2,3,3-tetrafluoropropoxy)phenyl]vinyl}-[1,2,4]triazol-1-yl)propyl]-1H-[1,2,4]triazol-4-ium bromide,
4-(4-acetoxy-3,5-dimethyl-benzyl)-1-[(2R,3R)-2-(2,4-difluorophenyl)-2-hydroxy-3-methanesulfonylbutyl]-1H-[1,2,4]triazol-4-ium bromide,
4-(4-Acetoxy-3,5-dimethylbenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxy-3-methylbenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxybenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxy-3,5-dichlorobenzyl)-1-[(2R-cis)-2-(2,4-difluorophenyl)-4-[4-[4-[4-[1-[(1S,2S)-(1-ethyl-2-hydroxypropyl)]-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxy-3-chlorobenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4- yl]phenyl]piperazin-1-yl]phenoxymethyl]tetrahydrofuran-2-ylmethyl]-1H-[1,2,4]triazol-4-ium bromide,
4-(4-acetoxy-3-methylbenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxybenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxy-3,5-dichlorobenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxy-3-chlorobenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxy-3,5-dimethylbenzyl)-1-[2-hydroxy-3-methyl-3-methylsulfanyl-2-(4-trifluoromethylphenyl)butyl]-1H-[1,2,4]triazol-4-ium bromide, and
4-(4-acetoxy-3,5-dimethyl-benzyl)-1-[[(5R,6R)-2-methoxyimino-3,3-dimethyl-6-[(4-trifluoromethoxyphenoxy)methyl]cyclohexyl]methyl]-1H-[1,2,4]triazol-4-ium bromide.

Example A:

Manufacture of dry ampoules for intramuscular administration:

A lyophilizate of 0.5 g 4-(4-acetoxy-3,5-dimethyl-benzyl)-1-[[(1R,6R)-2-methoxyimino-3,3-dimethyl-6-[(4-trifluoromethoxyphenoxy)methyl]cyclohexyl]methyl]-1H-[1,2,4]triazol-4-ium bromide is prepared in the usual manner and filled into an ampoule. Prior to the administration the lyophilizate is treated with 2.5 ml of a 2% aqueous lidocaine hydrochloride solution.

Example B:

Hard gelatin capsules each containing the following ingredients were manufactured in the conventional manner *per se*:

| | |
|---|---|
| 4-(4-Acetoxy-3,5-dimethyl-benzyl)-1-[[(5R,6R)-2-methoxyimino-3,3-dimethyl-6-[(4-trifluoromethoxyphenoxy)methyl]cyclohexyl]methyl]-1H-[1,2,4]triazol-4-ium bromide | 100 mg |
| Lactose | 56 mg |
| Crystalline Cellulose | 30 mg |
| Silicic acid, Light Anhydrous | 10 mg |
| Talc | 3 mg |
| Magnesium stearate | 1 mg |
| | Total 200 mg |

Example C:

Tablets each containing the following ingredients were manufactured in the conventional manner *per se*:

| | |
|---|---|
| 4-(4-Acetoxy-3,5-dimethyl-benzyl)-1-[[(5R,6R)-2-methoxyimino-3,3-dimethyl-6-[(4-trifluoromethoxyphenoxy)methyl]cyclohexyl]methyl]-1H-[1,2,4]triazol-4-ium bromide | 100 mg |
| Lactose | 60 mg |
| Corn starch | 20 mg |
| Sodium Starch Glycolate | 10 mg |
| Polyvinylpyrrolidone | 6 mg |
| Talc | 3 mg |
| Magnesium stearate | 1 mg |
| | Total 200 mg |

**Claims**

1. A compound of the general formula (I),

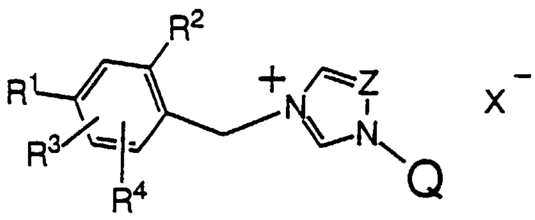

(I)

wherein

Q is the remainder of an azole compound of the formula II

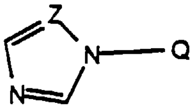

(II)

possessing antifungal activity;

Z is nitrogen or methine;
$R^1$ and $R^2$ are each independently a hydrogen atom or a group -OY [in which Y is a group easily hydrolyzable under physiological condition];
$R^3$ and $R^4$ are each independently a hydrogen or halogen atom, lower alkyl, lower alkoxy, lower alkylthio, (lower alkylcarbonyl)thiomethyl, carboxy or methoxycarbonyl; and
$X^-$ is a pharmaceutically acceptable anion,

as well as salts, hydrates or solvates of the compounds of the general formula (I).

**2.** The compound of claim 1, wherein the easily hydrolyzable group Y is the acyl residue of an amino acid or Y is selected from the group consisting of:

$$R^5CO\text{-, or } (R^6O)_2PO\text{-}$$

wherein $R^5$ is hydrogen, lower alkoxy, lower alkyl which may be optionally substituted with carboxyl, amino, lower alkyl amino, dilower alkyl amino, or aryl; and $R^6$ is hydrogen or lower alkyl.

**3.** The compound of claim 1 wherein

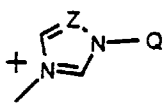

is a group derived from an azole compound of the group consisting of:

dl-1-[2-(2,4-dichlorophenyl)-2-[(2,4-dichlorophenyl)methoxy]ethyl]-1H-imidazole,
dl-cis-1-acetyl-4-[4-[2-(2,4-dichlorophenyl)-2-(imidazol-1-ylmethyl)-1,3-dioxolan-4-yl]methoxy]phenyl]piperazine,
dl-2-[(RS)-sec-butyl]-4-[4-[4-[4-[(2R,4S)-2-(2,4-dichlorophenyl)-2-(1H-1,2,4-triazol-1-ylmethyl)-1,3-dioxolan-4-yl]methoxy]phenyl]-1-piperazinyl]phenyl-3H-[1,2,4]triazol-3-one
2-[(1R,2R)-2-(2,4-diflorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone,
(+)-2-(2,4-difluorophenyl)-3-methyl-1-(1H-1,2,4-triazol-1-yl)-3-(6-(1H-1,2,4-triazol-1-yl)pyridazin-3-ylthio)butan-2-ol,
(2R)-2-(2,4-diflourophenyl)-1-[3-[(E)-4-(2,2,3,3-tetrafluoropropoxy)styryl]-(1,2,4-triazol-1-yl)-3-(1,2,4-triazol-1-yl)]propan-2-ol
dl-threo-2-(2,4-difluorophenyl)-3-methyl-sulfonyl-1-(1H-1,2,4-triazol-1-yl)-butan-2-ol,
(-)-4-[4-[4-[4-[[5-(2,4-difluorophenyl)-5-(1H-1,2,4-triazol-1-ylmethyl)tetrahydrofuran-3-yl]methoxy]phenyl]piperazinyl]phenyl]-2[(1S,2S)-1-ethyl-2-hydroxypropyl]-3H-1,2,4-triazol-3-one
(2R,3R)-3-[4-(4-cyanophenyl)thiazol-2-yl)]-2-(2,4-difluorophenyl)-1-(1H-1,2,4-triazol-1-yl)-butan-2-ol,
3-methyl-3-methylthio-1-(1,2,4-triazol-1-yl)-2-(trifluoromethylphenyl)-butan-2-ol,
1-[[(1R,2S,6R)-2-methoxy-3,3-dimethyl-6-(2-*p*-tolylethyl)cyclohexyl]methyl]-1H-[1,2,4]triazol, and
(5R,6R)-2,2-dimethyl-6-[(1H-1,2,4-triazol-1-yl)methyl]-5-[[4-(trifluoro-methoxy)phenoxy]methyl]cyclohexanone O-methyloxime.

**4.** A compound of claim 1 which is selected from the group consisting of:

dl-1-(4-acetoxy-3,5-dimethylbenzyl)-3-[2-(2,4-dichlorobenzyloxy)-2-(2,4-dichlorophenyl)ethyl]-3H-imidazol-1-ium bromide,
dl-1-(4-acetoxy-3-methylbenzyl)-3-[2-(2,4-dichlorobenzyloxy)-2-(2,4-dichlorophenyl)ethyl]-3H-imidazol-1-ium bromide,
dl-1-(4-acetoxybenzyl)-3-[2-(2,4-dichlorobenzyloxy)-2-(2,4-dichlorophenyl)ethyl]-3H-imidazol-1-ium bromide,
dl-1-(4-acetoxybenzyl)-3-[(2R,4S)-4-[4-(4-acetylpiperazin-1-yl)phenoxymethyl]-2-(2,4-dichlorophenyl)-[1,3]dioxan-2-ylmethyl]-3H-imidazol-1-ium bromide,
dl-1-(4-acetoxy-3,5-dimethylbenzyl)-3-[(2R,4S)-4-[4-(4-acetylpiperazin-1-yl)phenoxymethyl]-2-(2,4-dichlorophenyl)-[1,3]dioxan-2-ylmethyl]-3H-imidazol-1-ium bromide,
dl-1-(4-acetoxy-3-methylbenyl)-3-[(2R,4S)-4-[4-(4-acetylpiperazin-1-yl)phenoxymethyl]-2-(2,4-dichlorophenyl)-[1,3]dioxan-2-ylmethyl]-3H-imidazol-1-ium bromide,
dl-1-(4-acetoxy-3-methoxybenzyl)-3-[(2R,4S)-4-[4-(4-acetylpiperazin-1-yl)phenoxymethyl]-2-(2,4-dichlorophenyl)-[1,3]dioxan-2-ylmethyl]-3H-imidazol-1-ium bromide,
dl-3-[(2R,4S)-4-[4-(4-acetylpiperazin-1-yl)phenoxymethyl]-2-(2,4-dichlorophenyl)-[1,3]dioxan-2-ylmethyl]-1-(4-isobutyryloxy-3,5-dimethylbenzyl)-3H-imidazol-1-ium bromide,
dl-3-[(2R,4S)-4-[4-(4-acetylpiperazin-1-yl)phenoxymethyl]-2-(2,4-dichlorophenyl)-[1,3]dioxan-2-ylmethyl]-1-(4-pivaloyloxy-3,5-dimethylbenzyl)-3H-imidazol-1-ium bromide,
dl-3-[(2R,4S)-4-[4-(4-acerylpiperazin-1-yl)phenoxymethyl]-2-(2,4-dichlorophenyl)-[1,3]dioxan-2-ylmethyl]-1-[4-(2,2-dimethylpropionyloxy)benzyl]-3H-imidazol-1-ium bromide,
dl-4-(4-benzoyloxy-3,5-dimethylbenzyl)-1-[4-[4-[4-[4-(1-(2-butyl-5-oxo-1,5-dihydro-[1,2,4]triazol-4-yl)phenyl]piperazin-1-yl]phenoxymethyl]-2-(2,4-dichlorophenyl)-[1,3]dioxolan-2-ylmethyl]-1H-[1,2,4]triazol-4-ium bromide,
dl-4-[4-(pyridine-3-carbonyloxy)-3,5-dimethylbenzyl]-1-[4-[4-[4-[4-(1-(2-butyl-5-oxo-1,5-dihydro-[1,2,4]triazol-4-yl)phenyl]piperazin-1-yl]phenoxymethyl]-2-(2,4-dichlorophenyl)-[1,3]dioxolan-2-ylmethyl]-1H-[1,2,4]triazol-4-ium bromide,
dl-4-(4-acetoxy-3,5-dimethylbenzyl)-1-[4-[4-[4-[4-[1-(2-butyl-5-oxo-1,5-dihydro-[1,2,4]triazol-4-yl)phenyl]piperazin-1-yl]phenoxymethyl]-2-(2,4-dichlorophenyl)-[1,3]dioxolan-2-ylmethyl]-1H-[1,2,4]triazol-4-ium bromide,
dl-4-(4-acetoxy-3-methylbenzyl)-1-[4-[4-[4-[4-[1-(2-butyl-5-oxo-1,5-dihydro-[1,2,4]triazol-4-yl)phenyl]piperazin-1-yl]phenoxymethyl]-2-(2,4-dichlorophenyl)-[1,3]dioxolan-2-ylmethyl]-1H-[1,2,4]triazol-4-ium bromide,
dl-4-(4-acetoxybenzyl)-1-[4-[4-[4-[4-[1-(2-butyl-5-oxo-1,5-dihydro-[1,2,4]triazol-4-yl)phenyl]piperazin-1-yl]phenoxymethyl]-2-(2,4- dichlorophenyl)-[1,3]dioxolan-2-ylmethyl]-1H-[1,2,4]triazol-4-ium bromide,
dl-1-[4-(4-{4-[4-(1-sec-butyl-5-oxo-1,5-dihydro-[1,2,4]triazol-4-yl)-phenyl]-piperazin-1-yl}-phenoxymethyl)-2-(2,4-dichloro-phenyl)-[1,3]dioxolan-2-ylmethyl]-4-(4-hexanoyloxy-3,5-dimethylbenzyl)-1H-[1,2,4]triazol-4-ium methanesulfonate,
4-(4-acetoxy-3,5-dimethylbenzyl)-1-[(2R,3R)-2-(2,4-difluorophenyl)-2-hydroxy-3-{5-oxo-4-[4-(2,2,3,3-tetrafluoropropoxy)phenyl]-4,5-dihydro-[1,2,4]triazol-1-yl}butyl]-1H-[1,2,4]triazol-4-ium bromide,
4-(4-acetoxy-3,5-dimethylbenzyl)-1-[(2R)-2-(2,4-difluorophenyl)-2-hydroxy-3-methyl-3-(6-[1,2,4]triazol-1-yl-pyridazin-3-ylsulfanyl)butyl]-1H-[1,2,4]triazol-4-ium bromide,
4-(4-acetoxy-3,5-dimethylbenzyl)-1-[(2R)-2-(2,4-difluorophenyl)-2-hydroxy-3-(3-{(Z)-2-[4-(2,2,3,3-tetrafluoropropoxy)phenyl]vinyl}-[1,2,4]triazol-1-yl)propyl]-1H-[1,2,4]triazol-4-ium bromide,
4-(4-acetoxy-3,5-dimethylbenzyl)-1-[(2R,3R)-2-(2,4-difluorophenyl)-2-hydroxy-3-methanesulfonylbutyl]-1H-[1,2,4]triazol-4-ium bromide,
4-(4-acetoxy-3,5-dimethylbenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxy-3-methylbenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxybenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxy-3,5-dichlorobenzyl)-1-[(2R-cis)-2-(2,4-difluorophenyl)-4-[4-[4-[4-[1-[(1S,2S)-(1-ethyl-2-hydroxypropyl)]-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxy-3-chlorobenzyl)-1-[(2R-cis)-2-(2,4-difluorophenyl)-4-[4-[4-[4-[1-[(1S,2S)-1-ethyl-2-hydroxypropyl)-5-oxo-1,5-dihydro-[1,2,4]triazol-4-yl]phenyl]piperazin-1-yl]phenoxymethyl]tetrahydrofuran-2-ylmethyl]-1H-[1,2,4]triazol-4-ium bromide,
4-(4-acetoxy-3,5-dimethylbenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxy-3-methylbenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxybenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxy-3,5-dichlorobenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxy-3-chlorobenzyl)-1-[(2R,3R)-2-(2,4-difluorophenyl)-2-hydroxy-3-[4-(4-cyanophenyl)thiazol-2-yl]butyl]-1H-[1,2,4]triazol-4-ium bromide,
4-(4-acetoxy-3,5-dimethylbenzyl)-1-[2-hydroxy-3-methyl-3-methylsulfanyl-2-(4-trifluoromethylphenyl)butyl]-1H-[1,2,4]triazol-4-ium bromide,

4-(4-acetoxy-3,5-dimethylbenzyl)-1-[[(5R,6R)-2-methoxyimino-3,3-dimethyl-6-[(4-trifluoromethoxyphenoxy)methyl]cyclohexyl]methyl]-1H-[1,2,4]triazol-4-ium bromide, and

4-(4-acetoxy-3,5-dimethylbenzyl)-1-[(1R,2S,6R)-2-methoxy-3,3-dimethyl-6-(2-*p*-tolylethyl)cyclohexylmethyl]-1H-[1,2,4]triazol-4-ium bromide.

**5.** A compound of claim 1 which is selected from the group consisting of

(2R,3R)-4-(4-aminoacetoxy-2-carboxybenzyl)-1-[3-[4-(4-cyano-phenyl)thiazol-2-yl]-2-(2,4-difluorophenyl)-2-hydroxybutyl]-1H-[1,2,4]triazol-4-ium bromidetrifluoroacetic acid salt,

1-[(2R,3R)-3-[4-(4-cyanophenyl)-thiazol-2-yl]-2-(2,4-difluorophenyl)-2-hydroxybutyl]-4-[(S)-3,5-dimethyl-4-(pyrrolidine-2-carbonyloxy)-benzyl]-1H-[1,2,4]triazol-4-ium bromide trifluoroacetic acid salt,

(2R,3R)-4-(4-aminoacetoxy-3,5-dimethylbenzyl)-1-[3-[4-(4-cyano-phenyl)-thiazol-2-yl]-2-(2,4-difluorophenyl)-2-hydroxybutyl]-1H-[1,2,4]triazol-4-iumbromide trifluoroacetic acid salt,

(2R,3R)-4-[4-(3-aminopropionyloxy)-3,5-dimethylbenzyl]-1-[4-[4-(4-cyanophenyl)-thiazol-2-yl]-2-(2,4-dlfluorophenyl)-2-hydroxy-3-methylbutyl]- 1H-[1,2,4]triazol-4-ium bromide trifluoroacetic acid salt,

(2R,3R)-4-[4-(4-amino-butyryloxy)-3,5-dimethylbenzyl]-1-[4-[4-(4-cyano-phenyl)-thiazol-2-yl]-2-(2,4-difluorophenyl)-2-hydroxy-3-methylbutyl]-1H-[1,2,4]triazol-4-ium bromide trifluoroacetic acid salt,

(2R,3R)-4-[4-[[2-(aminoacetyl)amino]acetoxy]-3,5-dimethylbenzyl]-1-[3-[4-(4-cyanophenyl)-thiazol-2-yl]-2-(2,4-difluorophenyl)-2-hydroxy-butyl]-1H-[1,2,4]triazol-4-ium bromide trifluoroacetic acid salt,

1-[(2R,3R)-3-[4-(4-cyanophenyl)-thiazol-2-yl]-2-(2,4-difluorophenyl)-2-hydroxybutyl]-4-[4-[(S)-2,5-diaminopentoyloxy]-3,5-dimethyl-benzyl]-1H-[1,2,4]triazol-4-ium bromide trifluoroacetic acid salt,

4-[4-[(S)-2-amino-propionyloxy)-3,5-dimethylbenzyl]-1-[(2R,3R)-3-[4-(4-cyanophenyl)-thiazol-2-yl]-2-(2,4-difluorophenyl)-2-hydroxy-butyl]-1H-[1,2,4]triazol-4-ium bromide trifluoroacetic acid salt,

1-[(2R,3R)-3-[4-(4-cyanophenyl)-thiazol-2-yl]-2-(2,4-difluorophenyl)-2-hydroxybutyl]-4-[3,5-dimethyl-4-[(methylamino)acetoxy]benzyl]-1H-[1,2,4]triazol-4-ium bromide trifluoroacetic acid salt,

(2R,3R)-1-[3-[4-(4-cyanophenyl)-thiazol-2-yl]-2-(2,4-difluorophenyl)-2-hydroxybutyl]-4-[3,5-dimethyl-4-[(propylamino)acetoxy]benzyl]-1H-[1,2,4]triazol-4-ium bromide trifluoroacetic acid salt,

1-[(2R,3R)-3-[4-(4-cyanophenyl)-thiazol-2-yl]-2-(2,4-difluorophenyl)-2-hydroxybutyl]-4-[4-[(S)-2-(methylamino)propionyloxy]-3,5-dimethyl-benzyl]-1H-[1,2,4]triazol-4-ium bromide trifluoroacetic acid salt,

(2R,3R)-1-[4-[4-(4-cyanophenyl)-thiazol-2-yl]-2-(2,4-difluorophenyl)-2-hydroxy-3-methyl-butyl]-4-[3,5-dimethyl-4-[3-(methylamino)-propionyloxy]benzyl]-1H-[1,2,4]triazol-4-ium bromide trifluoroacetate,

4-[4-[(S)-2-amino-3-methyl-butanoyloxy]-3,5-dimethylbenzyl]-1-[(2R,3R)-3-[4-(4-cyanophenyl)-thiazol-2-yl]-2-(2,4-difluorophenyl)-2-hydroxybutyl]-1H-[1,2,4]triazol-4-ium bromide trifluoroacetic acid salt,

(2R,3R)-1-[3-[4-(4-cyanophenyl)-thiazol-2-yl]-2-(2,4-difluorophenyl)-2-hydroxy-butyl]-4-[4-[(isopropylamino)acetoxy]-3,5-dimethylbenzyl)-1H-[1,2,4]triazol-4-ium bromide trifluoroacetic acid salt,

1-[(2R,3R)-3-[4-(4-cyanophenyl)-thiazol-9-yl]-2-(2,4-difluorophenyl)-2-hydroxybutyl]-4-[(2S)-4-[2-(ethylamino)propionyloxy]-3,5-dimethyl-benzyl]-1H-[1,2,4]triazol-4-ium bromide trifluoroacetic acid salt,

(2R,3R)-1-[3-[4-(4-cyanophenyl)-thiazol-2-yl]-2-(2,4-difluorophenyl)-2-hydroxybutyl]-4-[4-[(ethylamino)acetoxy]-3,5-dimethylbenzyl]-1H-[1,2,4]triazol-4-ium bromide trifluoroacetic acid salt, and particularly

1-[2R,3R)-3-[4-(4-cyano-phenyl)-thiazol-2-yl]-2-(2,4-difluoro-phenyl)-2-hydroxy-butyl]-4-(3,5-dimethyl-4-methylaminoacetoxy-benzyl)-1H-[1,2,4]triazol-4-ium bromide hydrochloric acid salt.

**6.** A process for the manufacture of a compound of the general formula (I),

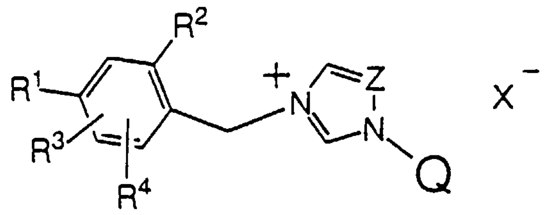

(I)

wherein $R^1$ to $R^4$, Q, $X^-$ and Z have the same meaning as in claim 1, as well as salts, hydrates of solvates thereof, which comprises reacting an azole compound possessing antifungal activity of the general formula (II),

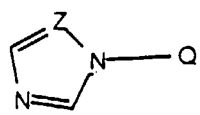

(II)

with a compound of the general formula (III),

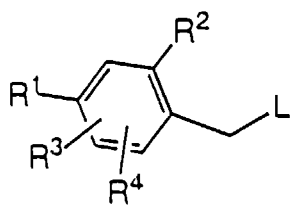

(III)

wherein $R^1$ to $R^4$ are the same as in claim 1 and wherein an amino group present in $R^1$ or $R^2$ may be in protected form ; and L is a leaving group.
followed if necessary, by removal of a protecting group and/or by salt formation.

**7.** A process as in claim 1 for the manufacture of a compound of claim 2, which comprises reacting a compound of the general formula (II) as defined in claim 6 with a compound of the general formula (III) as defined in claim 6, in which one of $R^1$ or $R^2$ is hydroxy, followed by reaction of the resulting compound with a compound of formula $R^5COL$, or $(R^6O)_2POL$, wherein $R^5CO$ is the acyl residue of a N-protected amino acid, or $R^5$ is hydrogen, lower alkoxy, lower alkyl which may be optionally substituted with carboxyl, amino, lower alkyl amino with or without protecting group, di-lower alkyl amino, or aryl; and $R^6$ is hydrogen or lower alkyl, and L is a leaving group.

**8.** An antifungal composition comprising a compound as defined in any one of claims 1 to 4 and a carrier.

**9.** A method for the therapy of fungal infections which comprising administering to the infected organism an effective amount of a compound as defined in any one of claims 1 to 4.

**10.** The use of a compound as defined in any one of claims 1 to 4 for the production of medicaments for the therapy of fungal infections.

**11.** The compound of claim 1 of the formula I',

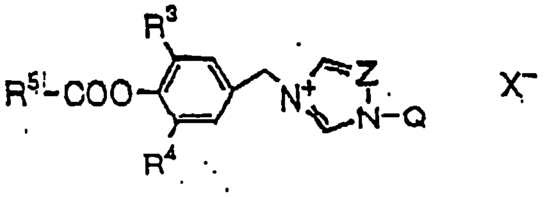
(I')

wherein

| | |
|---|---|
| $R^{5'}$ | is a straight-chain or branched $C_1$-$C_5$ alkyl, aryl, pyridyl, pyrrolidinyl or a group A-NH-B-(wherein A is a hydrogen atom or a straight-chain or branched $C_1$-$C_5$ alkyl; B is a straight-chain or branched $C_1$-$C_4$ alkylene, $-CH_2$-CONH-$CH_2$- or $-CH_2CH_2CH_2$-CH($NH_2$)-); |
| $R^3$ and $R^4$ | are each independently a hydrogen or halogen atom or a lower alkoxy; and |
| Z, Q and $X^-$ | are as defined in claim 1. |

**12.** A substituted azole compound of claim 1 or 2, wherein

Q is the group of the formula,

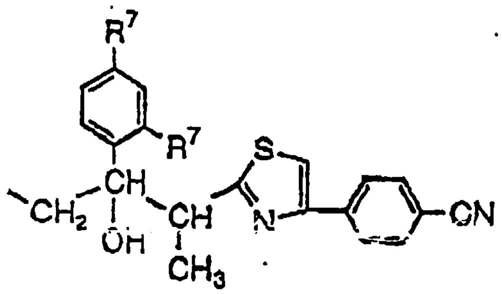
(Q1),

or

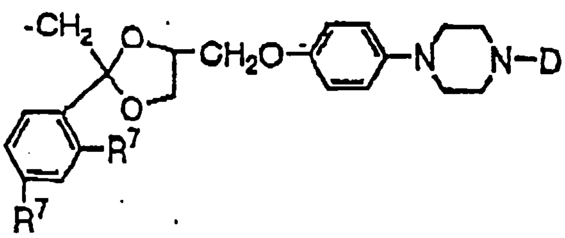
(Q2),

wherein

D is a lower alkanoyl or the group of the formula,

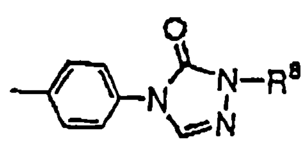

$R^7$ is a halogen atom; and
$R^8$ in a straight-chain or branched $C_1$-$C_4$ alkyl.